# EUROPEAN PATENT APPLICATION

(11) **EP 4 834 477 A1**
(43) Date of publication of application: **04.11.2026**
(21) Application number: 26190856.0
(22) Date of filing: 14.06.2022
(51) Int. Cl.: B01L 3/00

(54) **A MICROFLUIDIC EXTRACELLULAR ENVIRONMENT MONITORING DEVICE**

(30) Priority: 14.06.2021 EP 21179343
(62) Document number(s) of the earlier application(s) in accordance with Art. 76 EPC: 22733629.4 / 4 355 489
(71) Applicant: ShanX Medtech BV, 5612DH Eindhoven (NL)
(72) Inventor: Shanko, Eriola Sophia, Eindhoven (NL); van der Linden, Adrianus J, Eindhoven (NL)
(74) Representative: Arnold & Siedsma

(57) **Abstract**

A microfluidic extracellular environment monitoring device is provided comprising:
- a substrate comprising a microstructure formed therein, the microstructure comprising a channel and a sample chamber; and
- an opening in fluid communication with the microstructure;
wherein the opening is configured to load at least one sample solution into the microstructure and the microstructure is configured to induce flow of the at least one sample solution, and wherein at least one of the channel and the sample chamber comprises a part which is coated with a mixture, the mixture comprising at least the following components:
- a matrix element;
- a luminescent chemical sensor material configured to emit an emission spectrum dependent on extracellular environmental conditions in the microstructure;
wherein each of the components of the mixture is selected to be at least partially dissolvable or dispersible in the at least one sample solution so as to mix with the sample solution.

## Description

### FIELD OF THE DISCLOSURE

The present application relates to a microfluidic device, a system and method for extracellular environment monitoring, and to a use of a microfluidic device.

### BACKGROUND OF THE DISCLOSURE

Microbial infections have a large impact on public health. The agents that cause disease fall into five groups: viruses, bacteria, fungi, protozoa, and helminths (worms). Protozoa and worms are usually grouped together as parasites.

Infection does not necessarily lead to disease. Pathogens must first make contact with the host and then establish a focus of infection in order to cause infectious disease. To establish an infection, the pathogen must first colonize the skin or the internal mucosal surfaces of the respiratory, gastrointestinal, or urogenital tracts and then overcome or bypass the innate immune defenses associated with the epithelia and underlying tissues. If it succeeds, it will then provoke an adaptive immune response that will take effect after several days and will usually clear the infection. Pathogens differ greatly in their lifestyles and means of pathogenesis, requiring an equally diverse set of defensive responses from the host immune system.

Only when a microorganism has successfully established a site of infection in the host does disease occur, and little damage will be caused unless the agent is able to spread from the original site of infection or can secrete toxins that can spread to other parts of the body. In addition to clearing the infectious agent, an effective adaptive immune response prevents reinfection. For some infectious agents, this protection is essentially absolute, while for others infection is reduced or attenuated upon re-exposure.

Different infectious agents cause markedly different diseases, reflecting the diverse processes by which they damage tissues. Many extracellular pathogens cause disease by releasing specific toxic products or protein toxins, which can induce the production of neutralizing antibodies. Intracellular infectious agents frequently cause disease by damaging the cells that house them. The specific killing of virus-infected cells by cytotoxic T cells thus not only prevents virus spread but removes damaged cells. The immune response to the infectious agent can itself be a major cause of pathology in several diseases. The pathology caused by a particular infectious agent also depends on the site in which it grows; Streptococcus pneumoniae in the lung causes pneumonia, whereas in the blood the same bacteria causes a rapidly fatal systemic illness.

Infectious diseases can be devastating, and sometimes fatal, to the host. Many strategies are available to protect people from infection and to treat a disease once it has developed. Some are simple steps that individuals can take; others are national or global methods of detection, prevention, and treatment. An antimicrobial is an agent that kills microorganisms or stops their growth. Antimicrobial medicines can be grouped according to the microorganisms they act primarily against. For example, antibiotics are used against bacteria, and antifungals are used against fungi. They can also be classified according to their function. Agents that kill microbes are microbicides, while those that merely inhibit their growth are called bacteriostatic agents.

Bacteria can produce a new generation every 20 to 30 minutes, and viruses even faster. Because they reproduce so quickly, microorganisms can assemble in enormous numbers with great variety in their communities. If their environment suddenly changes, the community's genetic variations make it more likely that some will survive. This gives microbes a huge advantage over humans when it comes to adapting for survival. Rapid detection of an infection is, therefore, of crucial relevance in order to give the person the appropriate treatment as soon as possible. Prolonged sicknesses may result in an antimicrobial resistance (AMR) or in a worsening of the disease. Microbial resistance to antimicrobials is a growing concern mandating their prudent use.

Bacterial cell division is an exponential process. Monitoring the growth of a bacteria may include using a high-power microscope to image bacterial cells and using the imaging system to count the average number of bacteria. To sustain their growth, all bacteria rely upon a range of metabolic processes from which they derive energy. By-products of these reactions are secreted into the extracellular environment.

The growth of the bacteria can, therefore, also be determined by measuring the conditions of the extracellular environment. These conditions can refer to the pH, the O2 concentration, or the CO2 concentration. The extracellular environmental conditions can be measured using, for example a molecular or chemical indicator, which exhibit altered fluorescence emission spectra or intensity when exposed to changes in the conditions of the extracellular environment. This may bypass the need for high- resolution optics.

Devices based on the monitoring of microbial growth for detecting if a sample is infected are already known. Such devices may also be used for the determination of AMR (and especially resistance against antibiotics) within a sample, such devices being known as antimicrobial susceptibility testing (AST) devices. For example, monitoring bacterial growth in the presence of an antibiotic reveals the efficacy thereof. In the case of an ineffective antibiotic, bacterial growth will ensue, accompanied by the aforementioned extracellular environmental condition changes as well as an increase in the number of bacteria, whereas in the presence of effective antibiotics, bacterial growth will be hindered or will not occur.

AST may be used to probe for resistant phenotypes of pathogens and to determine a minimal dosage or minimal inhibitory concentration (MIC) of an antibiotic needed to inhibit the pathogen.

However, routine clinical tests for probing resistant or non-resistant pathogens in a subject may typically need two days to one week before receiving the results of AST from the time that the samples were collected. For example, the collected sample may need 24-48 hours of incubation before AST may be initiated. In the case of bacteremia and sepsis, a blood culture step may be needed with five days of incubation. Antimicrobial susceptibility testing may then take an additional 8-24 hours.

Thus, there is a need to have rapid testing devices for quickly and efficiently detecting an infection in a sample and/or identifying one or a plurality of antimicrobials to kill pathogens and a variety of metrics related to the identified one or a plurality of antibiotics to assist the health care professional. Rapid testing devices may prevent the need for administering large unnecessary doses of broad-spectrum antibiotics, reduce the emergence of AMR and the worsening of the disease.

WO 2018150414 A1 discloses methods and apparatus for fabricating and using AST kits based on microfluidic devices including an array of a plurality of chambers open to a primary channel in the microfluidic device. Each chamber is connected to secondary channel, also referred as an evacuation channel. The microfluidic devices according to WO2018150414 is also be known as stationary nanoliter droplet arrays (SNDA) since each of the plurality of chambers are configured to hold a volume of liquid also known as droplets on the order of nanoliters. This document further discloses a method for forming droplets with gradually varied concentrations of a solute in microfluidic device, with each of the droplets including one or a plurality of antibiotic solutes. According to WO 2018150414 A1, after fabricating AST kits with SNDA including a gradually varied concentration of antibiotics in each of the plurality of chambers, bacteria in a sample fluid is injected into primary channel and into each of chamber. Prior to introduction of the sample fluid into the chamber, the chamber is previously filled by a gas or by another fluid that is significantly less viscous than the sample fluid. The bacterial sample fluid is sealed by a retaining fluid (a substance of significantly lower surface tension) loaded into primary channel. Upon filling primary channel and each chamber with sample fluid, retaining fluid is loaded into primary channel purging bacterial sample fluid from primary channel. Then, at a second end of primary channel, only a retaining fluid is passed into secondary channel. In this manner, bacterial sample fluid droplets are completely sealed in each chamber by an immiscible barrier due to the retaining fluid in primary channel and secondary channel. WO2018150414 further discloses that a molecular or chemical indicator may be introduced in the bacterial sample fluid and subsequently into the sealed droplet, where a property of the indicator may change based on some input by the bacteria.

However, existing bacterial growth-based tests, like the microfluidic device according to WO 2018150414 A1, present some drawbacks and still need to be improved. Indeed, current bacterial growth- based tests require a long and multi-step preparation and experimental protocols and need to be carried out by qualified personnel.

For example, the AST disclosed in WO 2018150414 A1 requires, to monitor bacterial growth, either to pretreat the sample fluid, prior to its loading into the microfluidic device, by mixing it with a molecular or chemical indicator and to use a high-power microscope to image bacterial cells. Thus, such a microfluidic device needs to be carried out by qualified personnel which must be able to prepare the sample fluid with the relevant indicators prior their loading into the microfluidic device and to be able to manipulate a high-power microscope to image bacterial cells. In addition, the microfluidic device according to WO 2018150414 A1 is based on the SNDA technology which requires a specific qualification in order to well realize this rigorous multi-step experimental protocol. Indeed, the AST according to WO2018150414 further requires several subsequent steps in order to seal a sample fluid droplet into each chamber i.e. loading the sample fluid (premixed with the relevant indicators) together with a retaining fluid into primary channel, purging the sample fluid by loading again a retaining fluid into primary channel and sealing a sample fluid droplet in each chamber by loading a retaining fluid into secondary channel leading to an immiscible barrier due to the retaining fluid in primary channel and secondary channel. Finally, such microfluidic devices, like the one disclosed in WO 2018150414 A1, generate high costs related to the need to carry out the test by qualified personnel, to pretreat the sample fluid, to use a high-power microscope, to load retaining fluids, but also related to the long time required to perform such a multi-step experimental protocol.

In view of the above, there is a need to provide microfluidic devices that are improved. In particular, it is desirable to have microfluidic devices that are easier-to-use, user friendly as no sample preparation is needed, rapid and cost sensitive.

The inventors have surprisingly found that providing a microfluidic extracellular environment monitoring device comprising a microstructure being at least partially coated with a mixture comprising a matrix element and at least one luminescent chemical sensor emitting an emission spectrum dependent on extracellular environmental conditions overcome the drawbacks of prior art. The emission spectrum is dependent on the current extracellular environmental conditions, which can be altered for example by microbial behavior, thus altering the emission spectrum.

According to a first aspect, a microfluidic device for detecting the susceptibility of a microbe present in a sample solution is provided, preferably an antimicrobial susceptibility testing (AST) device is provided. The microfluidic device comprises:
- a substrate comprising a microstructure formed therein, the microstructure comprising a plurality of channels, wherein each channel and /or each sample chamber is coated with a mixture, the mixture comprising:
- a luminescent chemical sensor dissolvable or dispersible in the sample solution and configured to emit an emission spectrum dependent on extracellular environmental conditions in the microstructure;
wherein the mixture in at least one of the channels or chambers further comprises at least one predetermined drug, the at least one predetermined drug being an antimicrobial.

Considering AST devices like the one described in WO 2018 150 414 A1, the sensor may be designed to track extracellular changes as a proxy for microbial metabolism. In this way, microbes can be monitored with high sensitivity, as changes in the sensor's emittance spectrum are correlated to microbe presence, without the need to directly measure (and potentially penetrate or affect) the microbes themselves. Another advantage is that integration of a plurality of channels enables multiple AST experiments for determining the test sensitivity against various antimicrobial agents to be performed simultaneously and in a simple manner, i.e. on a single microfluidic device. A still further advantage is that a complex, multi-step protocol to carry out AST experiments can be dispensed, so that a more economic, faster and easier-to-use microfluidic device is provided. and easier-to-use PoC device to conduct AST, providing substantially faster results.

In embodiments of the present disclosure the components of the coating are selected such that the coated mixture is at least 40% or 50%, preferably at least 70%, more preferably at least 90% dissolvable or dispersible in the at least one sample solution. In embodiments of the present disclosure the components of the coated mixture are selected such that the mixture is substantially entirely or almost diffused to the sample solution within ten minutes, preferably within five minutes, even more preferably within one minute, after loading of the at least one sample solution into the microstructure.

In embodiments of the present disclosure the components of the coated mixture are selected to be dissolvable or dispersible in at least one sample solution comprising a water-based substance.

In embodiments of the present disclosure the water-based substance is a body fluid or fluid derived from a body fluid, the body fluid optionally being selected from the group consisting of urine, blood, lymph, saliva, wax, cerebrospinal fluid, milk and their mixtures, and/or the body fluid being obtained from a body tissue, the body tissue optionally being selected from the group consisting of dental tissues, connective tissues, such as cartilage, and their mixtures.

In embodiments of the present disclosure a further component of the mixture is a microbial growth agent, preferably the microbial growth agent being a bacterial nutrient, more preferably lysogeny broth, Mueller Hinton Broth and/or polysaccharides, wherein the microbial growth agent preferably is at least partially or fully dissolvable or dispersible in the sample solution.

In embodiments of the present disclosure a further component of the mixture is a predetermined drug, preferably the at least one predetermined drug being an antimicrobial, more preferably an anti-bacterial, still more preferably an antibiotic, wherein the predetermined drug preferably is least partially or fully dissolvable or dispersible in the sample solution.

In embodiments of the present disclosure a further component of the mixture is a mixing enhancer configured to enhance mixing of a dissolved or dispersed component by cooperation with a fully external manipulating platform.

In embodiments of the present disclosure the mixing enhancer comprises at least one entity which incorporates magnetic elements, the magnetic elements being configured to be moved by a magnetic field generated by the fully external manipulating platform, wherein preferably the at least one entity is a bead and wherein preferably the bead has a wall defining an internal volume wherein the magnetic elements are arranged.

The magnetic elements may be ferromagnetic elements, preferably ferrite magnetic material-based nanoelements, neodymium, alnico or samarium cobalt, more preferably iron oxide-based nanoelements. Furthermore, the at least one entity is coated with at least one biological recognition molecule, for example an antibody.

In embodiments of the present disclosure the at least one luminescent chemical sensor material is selected to move freely relative to microbes present in the sample solution when dissolved or dispersed in the sample solution.

In embodiments of the present disclosure the at least one luminescent chemical sensor material is arranged inside an entity and/or at a wall of an entity. When the sensor material is arranged inside the entity and in most cases also when the at least one luminescent sensor material is arranged at a wall of entity there is no direct contact with microbes present in the sample solution when dissolved or dispersed in the sample solution. However, in embodiments wherein the wall of the entity is antibody functionalized, the microbe may end up attached to the antibody and in this sense, there may be contact (albeit indirectly) with the luminescent material.

The fact that the present disclosure relates to a monitoring device which is aimed at an extracellular environment, implies that a luminescent chemical sensor material is selected that does not penetrate microbes present in the sample solution. This has the advantage that the microbes are not stained or, more generally, in any way affected in any way by the monitoring operation.

In embodiments of the present disclosure the microfluidic extracellular environment monitoring device comprises a plurality of the channels and associated sample chambers, each channel having a first end in fluid connection with an opening for loading the at least one sample solution and a second end in fluid connection with a sample chamber, wherein openings of the plurality of channels preferably form a single common opening for leading the at least one sample solution into each of the channels.

In embodiments of the present disclosure coated parts of each of the channels and/or sample chambers are coated by a mixture comprising a different predetermined drug.

In embodiments of the present disclosure the mixture comprises a plurality of different mixtures, the mixtures comprising different components and/or different concentrations of components.

In embodiments of the present disclosure at least one of the channel and sample chamber has a first part coated with a first mixture and second part coated with a second mixture, different from the first mixture.

In embodiments of the present disclosure only the first mixture comprises the chemical sensor material and/or only the second mixture comprises a microbial growth agent and/or a predetermined drug.

In embodiments of the present disclosure the sample chamber is at least partially transparent to the outside of the microstructure, wherein preferably substantially the entire substrate is transparent.

This may allow visual inspection of the sample solution inside the sample chamber and/or make it possible to measure the (variations in the) (intensity of the) light spectrum resulting from varying dependent on extracellular environmental conditions inside the sample chamber.

In embodiments of the present disclosure the coated part is present only in the channel. In this manner the mixture may be dissolved earlier and/or the risk of a non-dissolved portion of the mixture remaining in the microstructure influencing the measurement of the emission spectrum may be changed.

In embodiments of the present disclosure the coated part comprises a plurality of droplets of the mixture. This forms a so-called droplet coating. Additionally or alternatively, the coated part comprises a layer of the mixture. The coated part then forms a so-called layer coating.

In embodiments of the present disclosure the coated part comprises a powder, liquid, semi-liquid, semi-solid, and/or gel.

In embodiments of the present disclosure the mixture has a viscosity greater than water, preferably greater than or equal to 0.85 cP, more preferably greater than or equal to 1 cP, still more preferably greater than or equal to 1.3 cP, yet still more preferably greater than or equal to 1.8 cP, at a temperature of 30 °C as measured using a viscometer.

In embodiments of the present disclosure the luminescent sensing material is configured to emit an emission spectrum dependent on extracellular environmental conditions including at least one of pH and/or the concentration of O₂, CO₂, ammonia, calcium, magnesium, metals, lactate, cortisol, glucose, extracellular Adenosine Triphosphate (eATP) and/or polymers like glycans.

In embodiments of the present disclosure the at least one luminescent chemical sensor material is selected from the group consisting of quenchable fluorophores, thiols, ruthenium(II), osmium(II), rhenium(I), iridium(III), platinum(II) and palladium(II), rhodamine based dyes, indicators for Ca²⁺, indicators for Na⁺, indicators for K⁺, indicators for Cl⁻, potential-sensitive dyes, luciferase-based sensors and their mixtures.

In embodiments of the present disclosure the microstructure further comprises:
a secondary channel with a first end and a second end, the at least one sample chamber being opened to the first end of the secondary channel to enable a gas, preferably air, to exit the microfluidic extracellular environment monitoring device from the sample chamber and flow from the first end to the second end of the secondary channel,
the secondary channel optionally comprising a capillary stop arranged between the first end and second end of the secondary channel to prevent the at least one sample solution from exiting the at least one sample chamber.

In embodiments of the present disclosure the microstructure comprises a plurality of the substructures, each substructure comprising a channel with a first end and a second end, and a sample chamber in fluid communication with the channel, wherein the substructures are arranged in parallel, in series, or circularly, in the substrate to enable the at least one sample solution to flow into respective substructures along the associated channels from the first end directly to the second end of each channel into each of the plurality of sample chambers.

In embodiments of the present disclosure the sample chambers of the plurality of substructures are arranged on one or more concentric imaginary circles with respect to the opening.

In embodiments of the present disclosure the opening comprises a filter, preferably a filter membrane, to filter unwanted substances from the sample solution.

According to a second aspect an antimicrobial susceptibility testing (AST) system for detecting the susceptibility of a microbe present in a sample solution is provided. The system comprises:
- at least one microfluidic device as defined herein;
- a detecting device comprising a holder for holding the at least one microfluidic extracellular environment monitoring device, a light source configured to direct a light beam to the sample chamber, a light detector configured to detect light emitted from the sample chamber, and a spectrum analyzer unit configured to determine data representative of the varying emission spectrum of the detected emitted light resulting from the varying extracellular environmental conditions inside the sample chamber.

In embodiments of the present disclosure the system further comprises a manipulating platform (11) fully arranged outside the microfluidic extracellular environment monitoring device.

According to a third aspect a method for extracellular environment monitoring using a microfluidic device as defined herein, is provided, the method comprising:
- loading a sample solution into a microstructure through least one opening;
- inducing a capillary flow of the loaded sample solution from the at least one opening along at least one channel to at least one sample chamber;
- allowing the sample solution to contact with the mixture so as to at least partially dissolve the mixture in the sample solution;
- detecting light emitted from the sample chamber; and
- determining from the detected light a varying emission spectrum of the emitted light resulting from the varying extracellular environmental conditions inside the sample chamber.

Detecting light emitted from the sample chamber may comprise:
- directing a light beam from a light source to the sample solution inside the sample chamber;
- detecting light emitted from the sample chamber, the emitted light being caused by luminescence in the sample solution inside the sample chamber.

The method may further comprise, before allowing the sample solution to contact with the mixture:
monitoring extracellular environmental conditions in the at least one sample chamber, for collecting data corresponding to the emission spectrum of the at least one chemical sensor material.

The may also comprise periodically or substantially continuously monitoring extracellular environmental conditions in the at least one sample chamber, in real-time during the allowing the sample solution to contact with the mixture. For instance, upon loading the fluid sample into the microfluidic device, the fluid distribution within the channel(s) will already start to dissolve the matrix material. Consequently, when the user inserts the microfluidic device into the detecting device, the initial state/image of a dissolved matrix with the sensor will be obtained. Periodically, i.e. at fixed or varying time intervals, subsequent states and/or images may be obtained.

In embodiments of the present disclosure the capillary flow occurs during 0.1-10 seconds and/or wherein the monitoring is performed during 1-8 hours, preferably 3-5 hours.

In embodiment of the present disclosure the mixture is configured to be substantially diffused to or dissolved in the sample solution within 10 minutes, preferably within 5 minutes, even more preferably within 1 minute, after loading of the at least one sample solution into the microstructure.

In embodiments of the present disclosure at least partially dissolving the mixture (7) comprises dissolving at least 40 or 50%, preferably at least 70%, more preferably at least 90% of the mixture, and most preferably comprises dissolving substantially all of the mixture.

In embodiments of the present disclosure the method further comprises enhancing the mixing of the sample solution with the mixture by applying a magnetic field within the at least one sample chamber to move a magnetic elements present in the matrix.

In embodiments of the present disclosure the method further comprises enhancing the mixing of the sample solution with the mixture by applying ultrasound to the sample chamber or by other active (micro)mixing enhancement methods. Alternatively or additionally, the enhancement could be accomplished by passive mixing in the channel. One way of accomplishing passive mixing is when the design of the channel is aimed to cause the solution flowing along the channel to mix. An example of such design is formed by a so-called staggered herringbone mixer although other types of channel designs with mixing effect are possible as well.

According to further aspects the present disclosure relates to the use of a microfluidic device for detecting a microbe present in a sample solution and/or to the use of a microfluidic extracellular environment monitoring device for detecting the susceptibility of a microbe present in a sample solution to a predetermined drug and/or for detecting the relative susceptibility of a microbe present in a sample solution to a plurality of different predetermined drugs.

### BRIEF DESCRIPTION OF THE DRAWINGS

In order for the present disclosure, to be better understood and for its practical applications to be appreciated, the following Figures are provided and referenced hereafter. It should be noted that the Figures are given as examples only and in no way limit the scope of the disclosure. Like components are denoted by like reference numerals.

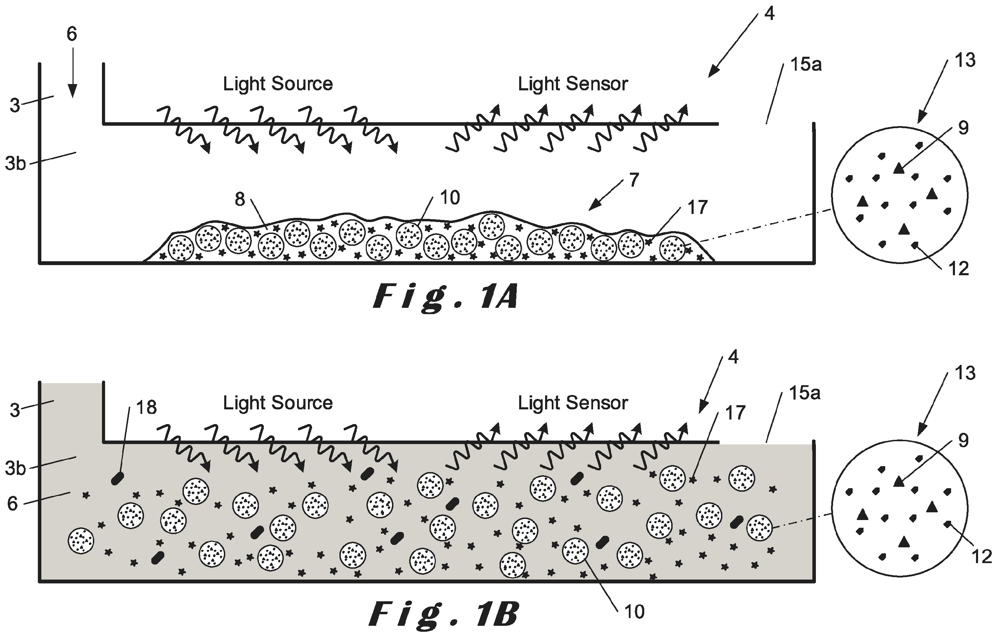

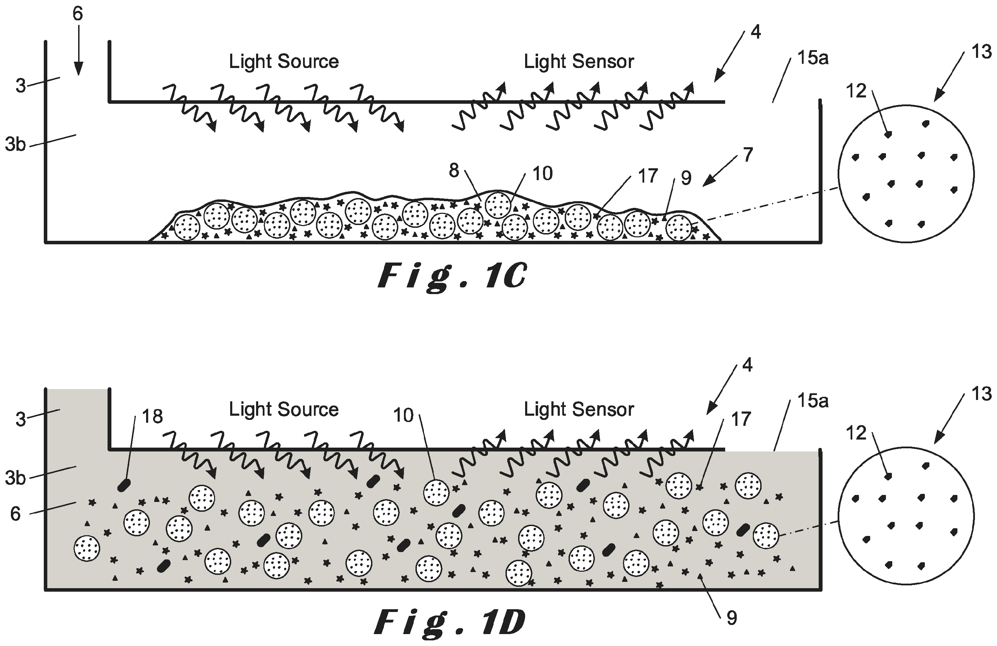

Figures 1a, 1b, 1c and 1d: Schematic representations of particular embodiments of the mixture 7 coating at least partially a sample chamber 4, for instance a culture chamber, prior the loading of the sample solution 6 (Figures 1a and 1c) and dissolved, post (Figures 1b and 1d) injection of the sample solution 6. Prior to sample injection 6, the components required for the measurement and testing are present in the chamber 4, in in the mixture coating. Upon administration of the sample 6 these components will dissolve or disperse potentially aided by mixing in the solution 6, mixing can be initiated and luminescence measurements can be performed.

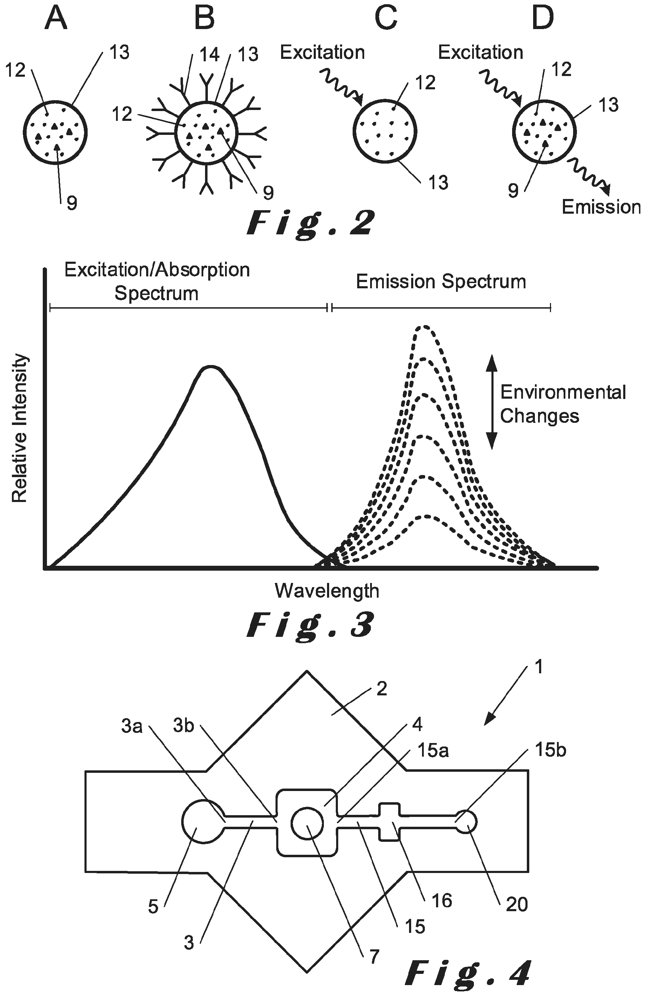

Figure 2: Schematic representation of particular embodiments of the luminescent chemical sensors 9. A) A bead 13 incorporating magnetic nanoelements 12 and luminescent chemical sensors 9 (i.e. functionalization) to sense extracellular environmental conditions. B) The luminescent chemical sensors 9 is identical to A), with the addition of an antibody 14 on the bead surface 13. C) Non-functionalized beads 13 (i.e. not incorporating luminescent chemical sensors 9) will not provide fluorescence readouts when stimulated with an excitation source. D) Bead (13) is identical to (C) but functionalized (i.e. incorporating) with the chemical luminescent sensor 9 allowing the bead 13 to provide a fluorescent readout when excited due to the inclusion of the luminescent chemical sensor 9. The emission signal can vary in accordance with extracellular environmental conditions (i.e. sensing).
Figure 3: Graphic representation of a possible change in readout from the luminescent chemical sensor 9 either free (i.e. about) or incorporated in (i.e. functionalized) a bead 13 in a culture chamber 4 of the microfluidic device 1 according to the present disclosure. The luminescent chemical sensor 9 can be excited at a specific wavelength based on the excitation spectrum (solid line) of the relevant sensor 9. The current extracellular environmental conditions will determine the emission spectrum (dashed lines) of the chemical sensor 9. The preferred behavior of the chemical sensor 9 is a change in intensity of the emission spectrum, which can be directly correlated to changes of the conditions in the extracellular environment ( e.g. changes in the pH, O2 concentration, and/or CO2 concentration).
Figure 4: schematic illustration of a microfluidic device 1 comprising one culture chamber 4 in accordance with some embodiments of the present disclosure.
Figure 5: schematic illustration of a microfluidic device 1 in accordance with some embodiments of the present disclosure, said microfluidic device 1 being represented in an exploded view.
Figure 6: schematic illustration of a microfluidic device 1 in accordance with some embodiments of the present disclosure.
Figure 7: external view of a readout device in accordance with some embodiments of the present disclosure.
Figures 8a; 8b; 8c; 8d; 8e; 8f; 8g: cross-sections of a readout device in accordance with some embodiments of the present disclosure.

The present disclosure will be described in the following with respect to particular embodiments and with reference to certain drawings, but the disclosure is not limited thereto.

Unless defined otherwise, all technical and scientific terms used herein have the same meaning as commonly understood by one of ordinary skill in the art to which the disclosure pertains. Although any methods and materials similar or equivalent to those described herein may be used in the practice for testing of the present disclosure, the preferred materials and methods are described herein.

It is also to be understood that the terminology used herein is for the purpose of describing particular embodiments only, and is not intended to be limiting.

Although embodiments of the disclosure are not limited in this regard, the terms "plurality" and "a plurality" as used herein may include, for example, "multiple" or "two or more". The terms "plurality" or "a plurality" may be used throughout the specification to describe two or more components, devices, elements, units, parameters, or the like. Unless explicitly stated, the method embodiments described herein are not constrained to a particular order or sequence. Additionally, some of the described method embodiments or elements thereof can occur or be performed simultaneously, at the same point in time, or concurrently. Unless otherwise indicated, use of the conjunction "or" as used herein is to be understood as inclusive (any or all of the stated options).

As described herein before and as shown in Figure 4, the present disclosure provides a microfluidic extracellular environment monitoring device 1 comprising:
a microstructure 2 formed in a substrate comprising a primary channel 3 with a first end 3a and a second end 3b, and at least one culture chamber 4 that opens to said primary channel 3; and at least one opening 5 coupled to said first end 3a of said primary channel 3, to load at least one sample solution 6 into said microstructure 2 through said first end 3a of said primary channel 3 to flow along said primary channel 3 from said first end 3a to said second end 3b into said at least one culture chamber 4;
wherein said microstructure 2 is at least partially coated with a mixture 7 comprising a matrix element 8 and at least one luminescent chemical sensor 9 emitting an emission spectrum dependent on extracellular environmental conditions.

A luminescent chemical sensor 9 emits an emission spectrum dependent on the extracellular environment. This spectrum may change for example as a microbe alters the extracellular environment.

Furthermore, the emission spectrum may also remain identical in the event of changes to the extracellular environment, however the intensity of this emission may be altered (i.e. reduced/increased depending on the environmental change) (Figure 3).

The inventors have surprisingly found that a microfluidic device 1 according to the present disclosure, wherein said microstructure 2 is at least partially coated with a mixture 7 comprising a matrix element 8 and at least one luminescent chemical sensor 9 emitting an emission spectrum dependent on extracellular environmental conditions, is improved compared to prior art devices since the microfluidic device 1 according to certain embodiments of the present disclosure may be easier-to-use, rapid and cost sensitive.

Indeed, since said mixture 7 comprising a matrix element 8 and at least one luminescent chemical sensor 9 emitting an emission spectrum dependent on extracellular environmental conditions is already located inside the microstructure 2 (e.g. in the primary channel 3 and/or the culture chamber 4) as a coating, prior to the loading of the sample solution 6, no preparation protocol is needed. Contrary to the microfluidic device according to WO2018150414 wherein a specialized person has to prepare the sample fluid with the relevant indicators prior their loading into the microfluidic device, the microfluidic device 1 according to the present disclosure allows to cancel this pretreatment step and to directly load the sample solution into the culture chamber 4 through the opening 5 coupled to the primary channel 3.

Also, the coating of said mixture 7 comprising a matrix element 8 and at least one luminescent chemical sensor 9 emitting an emission spectrum dependent on extracellular environmental conditions within the microstructure 2 (e.g. in the primary channel 3 and/or the culture chamber 4) allows to test extracellular environment by bypassing the need for high-resolution optics.

In addition, the experimental protocol may be done in a single-step manner. Indeed, the experimenter has only to load the sample solution 6 into the culture chamber 4 through the opening 5 coupled to the primary channel 3, contrary to the experimental protocol disclosed in WO2018150414 based on the SNDA technology which requires a rigorous multi-step experimental protocol. Indeed, the AST according to WO2018150414 requires several subsequent steps in order to seal a sample fluid droplet into each chamber i.e. loading the sample fluid (premixed with the relevant indicators) together with a retaining fluid into primary channel, purging the sample fluid by loading again a retaining fluid into primary channel and sealing a sample fluid droplet in each chamber by loading a retaining fluid into secondary channel leading to an immiscible barrier due to the retaining fluid in primary channel and secondary channel.

Such a multi-step experimental protocol, as disclosed in WO2018150414, can only be performed by someone who received a specific qualification. On the contrary, the use of the microfluidic device 1 for extracellular environment monitoring according to the present disclosure is very easy-to-use. Indeed, the experimenter has only to load the sample solution, without a pretreatment step, into the culture chamber 4 through the opening 5 coupled to the primary channel 3. Therefore, according to certain embodiments, no specific qualification is required to perform extracellular environment monitoring and the microfluidic device 1 can be used, for example, by a doctor, a nurse, a pharmacist or a military.

Finally, the microfluidic device 1 according to the present disclosure allows to rapidly monitor extracellular environment in a sample solution. In particular, the microfluidic device 1 according to the present disclosure allows to monitor extracellular environment only in less than 8 hours, preferably in less than 6 hours, more preferably in less than 4 hours, from sample collection until readout. Such a rapid monitoring of extracellular environment using the microfluidic device 1 of the present disclosure prevents the need for administering large unnecessary doses of broad spectrum antimicrobials, reduces the emergence of AMR and prevents the worsening of the disease. Existing routine clinical tests as well as known bacterial growth-based microfluidic devices, such as disclosed in WO2018150414, need more time before receiving the results on extracellular environment at least because of the requirement of a pretreatment protocol and a rigorous multi-step experimental protocol (a premix of the sample fluid with indicators and several loadings of retaining fluids, as disclosed in WO2018150414).

Therefore, the microfluidic device 1 according to the present disclosure is easier-to-use and allows rapid extracellular environment monitoring i.e. in less than 8 hours, preferably in less than 6 hours, more preferably in less than 4 hours, from sample collection until readout. In particular, no premix (prior to their loading) of the sample solution with indicators is needed, the use of a high power microscope is not mandatory, the experimental protocol can be performed in a single-step manner i.e. by loading only the sample solution to be tested without the need to load one or more retaining fluids. Furthermore, the reduction of required reagents (e.g. retaining fluid), the skip of the premix step, the simplified experimental protocol (unique loading of the biological sample solution) and the non-necessity of specialized personnel to perform the test, allow to provide a microfluidic extracellular environment monitoring device which is cost sensitive.

The mixture 7 according to the present disclosure at least partially coating said microstructure 2 and comprising a matrix element 8 and at least one luminescent chemical sensor 9 emitting an emission spectrum dependent on extracellular environmental conditions, will dissolve in the sample solution 6 when loaded into said microstructure 2 through said first end 3a of said primary channel 3 to flow along said primary channel 3 from said first end 3a to said second end 3b into said at least one culture chamber 4.

In a preferred embodiment, said mixture 7 coating at least partially said microstructure 2 is present in the form of a droplet or of a layer.

In a particular embodiment of the present disclosure, a volume comprised between 0.5 and 5 µl of said mixture 7 is deposited as a coating on said microstructure 2, preferably a volume comprised between 0.5 and 2 µl, more preferably comprised between 0.8 and 1.5 µl, still more preferably of about 1 µl.

In a more preferred embodiment, said mixture 7 is a coating, preferably a partial coating.

In a particular embodiment, said mixture 7 is a droplet coating.

In another particular embodiment, said mixture 7 is a layer coating. For example, said mixture 7 is a film-like coating.

As used herein, the term "coating" may refer to the covering of a surface with a substance. A coating can be total i.e. the substance covers the entire surface, or partial i.e. the substance covers a part of the surface. A partial coating leads to a surface being at least partially coated with a substance.

In some embodiments, said coating is performed by a deposition process. The deposition process can be either physical or chemical, whereby evaporation and sputtering techniques form the former and chemical vapor deposition and sol-gel technique comprise the latter.

According to certain embodiments of the present disclosure, the deposition process comprises the following steps:
a drop of mixture 7 is applied in the center of the culture chamber 4;
the drop is dispersed, preferably with a pipette tip, across the whole chamber 4 resulting to a thin layer of the coating material 7; and
the mixture 7 is left to dry, preferably partially, in room temperature for 3 hours.

In some embodiments of the present disclosure, said mixture 7 is in the form of a droplet coating a part of said microstructure 2.

In some embodiments of the present disclosure, said mixture 7 is in the form of a layer coating a part of said microstructure 2.

In a more preferred embodiment, said mixture 7 coating at least partially said microstructure 2 is present in said primary channel 3 and/or in said at least one culture chamber 4.

When said mixture 7 is present in said at least one culture chamber 4, either as a drop or as a layer, it will dissolve in the sample solution 6 when loaded through said at least one opening 5 and flowing from said first end 3a to said second end 3b of said primary channel 3 into said at least one culture chamber 4 (Figure 1a; 1b, 1c and 1d).

When said mixture 7 is present in said at least one primary channel 3, either as a drop or as a layer, it will dissolve in the sample solution 6 when loaded through said at least one opening 5 and flowing from said first end 3a to said second end 3b of said primary channel 3 into said at least one culture chamber 4.

In a particular embodiment, said mixture 7 is present in said primary channel 3. Inventors have surprisingly observed an accelerated diffusivity of said mixture 7 in the sample solution 6, when said primary channel 3 is at least partially coated with said mixture 7. According to this particular embodiment, inventors have found that the readout is improved (more sensible) and more rapid. Also, when said mixture 7 is present in said primary channel 3, the need of a mixing enhancer 10 is reduced.

In a particular embodiment, said mixture 7 is present in said at least one culture chamber 4.

In some embodiments, said mixture 7 is in the form of a droplet coating a part of said primary channel 3 and/or a part of said culture chamber 4.

In some embodiments, said mixture 7 is in the form of a layer partially or totally coating said primary channel 3 and/or a part of said culture chamber 4.

In a preferred embodiment, said matrix element 8 is a polymer. In a more preferred embodiment, said matrix element is a polymer selected from the group consisting of polyols such as glycerol or glycerin, hydrogel, sugar, monomers such as amino acids, methylamines, methylsulfonium compounds, (poly)ethylene glycol, and their mixtures.

For example, said luminescent chemical sensor 9 is mixed with said matrix element 8, for example glycerol, obtained said mixture 7 being deposited as a coating either on the bottom or on the top of said at least one culture chamber 4.

Said mixture 7 can be left to dry or seal said microfluidic device 1 immediately.

The term "bottom" within the meaning of the present disclosure corresponds to the lower wall of said culture chamber which is intended to rest on its support, for example a table. The term "top" within the meaning of the present disclosure correspond to the wall opposite to the bottom i.e. the lower wall of said culture chamber.

In a particular embodiment, said mixture 7 can be deposited as a coating both on the bottom and/or on the top of said at least one culture chamber 4.

In another particular embodiment, said mixture 7 further comprises nutrients (not represented), preferably LB (lysogeny broth) nutrients.

In a more preferred embodiment of the present disclosure, said mixture 7 has a viscosity greater than water at 30 °C.

In a still more preferred embodiment, said mixture 7 has a viscosity greater than or equal to 0.85 centipoise (cP), preferably greater than or equal to 1 cP, more preferably greater than or equal to 1.3 cP, still more preferably greater than or equal to 1.8 cP at a temperature of 30 °C using a viscometer.

The viscometers may be the No. 50, 100, 200, 300 sizes of the modified Ostwald viscometers described by Cannon and Fenske. Specifications and directions for use are given by the American Society for Testing Materials (ASTM), especially in ASTM D446 and ASTM D445.

In still a more preferred embodiment, said matrix element 8 is glycerol.
In some embodiment, said mixture 7 comprises from 1 to 50 percent by weight (wt.%) of said matrix element 8, preferably from 2 to 30 wt.%, more preferably from 5 to 25 wt.%, more preferably from 10 to 25 wt.%, still more preferably of about 20 wt.%. As used herein, the term "wt. %" refers to "percent by weight", also called "percent by mass" or sometimes also written as "w/w", which is defined as the percent of the total mass of the solution that is one component. For example, 2 wt. % of matrix element means that the mixture comprises 2 g of said matrix element for every 100 g of the mixture."%" or "percentage" refers to percent by weight, unless the context wherein it is mentioned provides a different meaning.

In a particular embodiment, mixture 7 comprises from 1 to 50 wt.% of glycerol, preferably from 5 to 30 wt.%, more preferably from 10 to 30 wt.%, more preferably from 10 to 25 wt.%, more preferably from 10 to 20 wt.%, still more preferably about 10 wt.%. In some embodiments the mixture 7 comprises from 10 to 30 wt.% of glycerol and has a viscosity comprised between 1 and 1.9 cP at 30 °C measured with a viscometer. In some embodiments the mixture 7 comprises from 10 to 20 wt.% of glycerol and has a viscosity comprised between 1 and 1.4 cP at 30 °C measured with a viscometer. In some embodiments the mixture 7 comprises about 20 wt.% of glycerol and has a viscosity of 1.35 cP at 30 °C measured with a viscometer. In some embodiments the mixture 7 comprises about 10 wt.% of glycerol and has a viscosity of 1.03 cP at 30 °C measured with a viscometer.

Viscosity of glycerol is measured according to the methodology described in the publication of J.B. Segur and Helen E. Oberstar, Viscosity of glycerol and its aqueous solutions (The Miner Laboratories, Chicago 6, III, Industrial and Engineering chemistry, September 1951, pages 2117-2120, Vol. 43, No. 9), its content being herein fully incorporated by reference. The used viscometers may be the No. 50, 100, 200, 300 sizes of the modified Ostwald viscometers described by Cannon and Fenske. Specifications and directions for use are given by the American Society for Testing Materials (ASTM), especially in ASTM D446 and ASTM D445.

According to another particular embodiment of the present disclosure the mixture 7 is present in a solid form or in a semi-solid form. As used herein, the term "semi-solid" refers to substances that have properties intermediate between those of a solid and a liquid and that are able to flow, but not completely freely. Semi-solid substances may also be referred as semi-liquid substances, both "semi-liquid" and "semi-solid" terms being used herein interchangeably.

In a preferred embodiment the mixture 7 is dried. A microfluidic device according to the present disclosure wherein said mixture 7 is dried, may be stored for longer period of time.

In a more preferred embodiment, the drying of the mixture 7 is performed by evaporation or by lyophilization. Lyophilization may also be referred as freeze-drying, both "lyophilization" and "freeze-drying" terms being used herein interchangeably. To lyophilize the mixture 7, the mixture 7 may be frozen, for example, at -80 °C for 40 minutes, and may be then subsequently placed into vacuum chambers for overnight lyophilization in a lyophilizer machine. To evaporate the mixture 7, said mixture 7 is exposed to air or vacuum.

In a more preferred embodiment, said mixture 7 is in the form of a powder or of a gel.

In a still more preferred embodiment, said mixture 7 is prefilled, i.e. is present in said microstructure 2 , e.g. in said primary channel 3 and/or in said at least one culture chamber 4, prior to the loading of said sample solution 6 within said at least one culture chamber 4 through said at least one opening 5.

In another preferred embodiment, said microfluidic device 1 includes a protective film (not represented) for sealing the device 1. Said protective film may be penetrable or removable from the microstructure 2. In some embodiments, the microstructure 2 and/or said protective film may be transparent to enable viewing the interior of microfluidic device 1. Typically, said protective film is removed just before use of the microfluidic device 1, e.g. just before loading the sample solution 6.

In a particular embodiment said protective film may be penetrable allowing a pinch in said at least
one opening 5 and/or said inlet 5a to load the sample 6.

In another particular embodiment, said protective film may surround said device 1 as a packaging.

In still another particular embodiment, said protective film is penetrable and surround said device
1.

In a particular embodiment, said at least one opening 5 enables fluids (e.g. air, sample solution 6) to be introduced into or removed from the interior of the microfluidic device 1 such as from the tip of a pipette, for example.

The present disclosure provides a microfluidic extracellular environment monitoring device 1 comprising a microstructure 2 formed in a substrate. The substrate may be made from various materials.

For example, the substrate may be made from a polymer, such as polydimethylsiloxane (PDMS or dimethicone), or another suitable polymer or material, elastomers, polymers, paper, thermoplastics, hydrogels, thermosets or glass/ silicon. For example, the substrate may be made from a thermoplastic material, such as poly(methylmethacrylate) (PMMA), Polycarbonate, Cyclic olefin copolymer (CoC), paper, or NOA81.

Said microstructure 2 formed in a substrate may be constructed of a distinct material from said protective film, for example, said microstructure 2 are prefabricated and attached to said protective film.

Said device 1 may be manufactured in various manufacturing processes, such as photolithography, laser, micro-EDM and micromechanical machining (micro-cutting and micromilling), injection molding, or thermal chip fabrication techniques.

Said device 1 may be manufactured using other or additional processes. Microstructures 2 may be single-tiered or multi-tiered. The microstructural patterns may be configured to provide functionality for microfluidic device. Different microstructural patterns may be employed with one or a plurality of microfluidic devices depending on the nature of the sample, reagent or other fluids intended to be used with microfluidic device. Different microstructural patterns may be used with microfluidic device, e.g. depending on the external environment of microfluidic device or other criteria.

According to a particular embodiment, said device 1 is manufactured by injection molding.

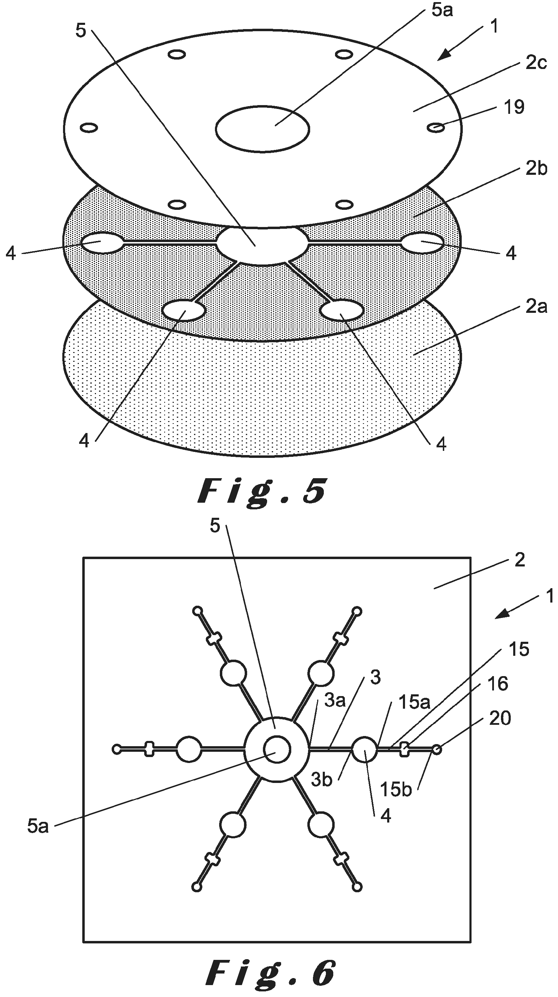

Figure 5 shows an exploded view of an embodiment of the microfluidic device 1 obtained according to another particular manufacturing process. According to such a embodiment, the substrate of the device 1 is fabricated by laser cutting a number of transparent PMMA sheet-like layers. Preferably, the substrate comprises three layers: a bottom layer 2a, a middle layer 2b and a top layer 2c. The microstructure 2 is arranged in at least one of the layers (in the embodiment actually shown in figure 5 the microstructure 2 is formed in two of the three layers). Double-sided tape or glue is attached on the bottom and the top sides of the middle layer 2b to allow adhesion of all the layers 2a, 2b, 2c together.

Preferably, said bottom layer 2a has a thickness comprised between 0.5 and 5 mm, more preferably comprised between 0.8 and 2 mm, still more preferably comprised between 1 and 1.5 mm, still more preferably, of about 1 mm.

In some embodiments, said middle layer 2b has a thickness comprised between 0.2 and 2 mm, preferably comprised between 0.3 and 1.5 mm, more preferably comprised between 0.25 and 1 mm, still more preferably of about 0.5 mm.

In some embodiments, said middle layer 2b when said double-sided tape is applied has a thickness comprised between 0.5 and 2.5 mm, preferably, comprised between 0.8 and 2 mm, more preferably between 0.8 and 1.5 mm, still more preferably of about 0.82 mm.

In a preferred embodiment of the present disclosure, said top layer 2c has a thickness comprised between 0.2 and 2 mm, preferably comprised between 0.3 and 1.5 mm, more preferably comprised between 0.5 and 1 mm, still more preferably of about 0.5 mm.

Preferably, said bottom layer 2a is arranged to hold said layers 2a, 2b and/or 2c together. More preferably, said middle layer 2b is arranged to allow the distribution of said sample solution 6 into said at least one culture chamber 4.

According to certain embodiments of the present disclosure, said middle layer 2b comprises the microstructural patterns of said microstructures 2, e.g. said at least one opening 5, said primary channel 3 and/or said at least one culture chamber 4. Preferably, said top layer 2c comprises an inlet 5a. In some embodiments, said top layer 2c and said middle layer 2b are arranged to allow said inlet 5a and said at least one opening 5 to stack (be aligned) in such a manner that a fluid, e.g. air and/or said sample solution 6, is able to flow from said inlet 5a into said at least one opening 5. In some embodiments of the present disclosure, said top layer 2c comprises an outlet 19 to enable a gas, preferably air, to exit the device 1 from said at least one culture chamber 4.

According to certain embodiments of the present disclosure, preferably when said microstructure 2 comprises said 3 layers (i.e. said bottom layer 2a, said middle layer 2b and said top layer 2c), said deposition process comprises the following steps:
a drop of mixture 7 is applied in the center of the culture chamber 4;
the drop is dispersed, preferably with a pipette tip, across the whole chamber 4 resulting to a thin layer of the coating material 7;
the mixture 7 is left to dry, preferably partially, in room temperature for 3 hours; and
said top layer 2c is placed atop.

Said microstructure 2 may have had surface treatment to hydrophilize or hydrophobize of the middle layer 2b i.e. said at least one opening 5 and/or said secondary opening 20.

In some embodiments of the present disclosure, said microstructure 2 may comprises 2 layers.

In a preferred embodiment, the microfluidic device 1 may include a plurality of microstructures 2 (not represented). All the preferred and particular embodiments of the microfluidic extracellular environment monitoring device 1 according to the present disclosure apply identically to a microfluidic extracellular environment monitoring device 1 including a plurality of microstructures 2.

Said microstructure 2 or said plurality of microstructures 2 may include channels, pumps, valves, mixers, chambers, vents or other components in a microfluidic device.

Said at least one opening 5 is coupled to said first end 3a of said primary channel 3 to load at least one sample solution 6 into said at least one culture chamber 4. For example, a biological sample solution 6 may be injected into said opening 5 that connects either directly or indirectly (e.g. via an intervening channel) to primary channel 3.

In a particular embodiment, said at least one opening 5 may comprise a filter, for example, to separate plasma from blood or impurities from urine.

Prior to introduction of the sample solution 6 into said culture chamber 4, the culture chamber 4 may have previously been filled by a gas (e.g. air) or vacuum. For example, the microfluidic device 1, prior to filling with said sample solution 6, may have kept in a controlled atmosphere or environment from which air was excluded.

In some embodiments of the present disclosure, said microstructure 2 may include 1 to 6 or more culture chambers 4, for example, where each culture chamber 4 may hold a fluid volume equal or less than 30 µl, preferably equal or less than 20 µl.

In a particular embodiment of the present disclosure, said microstructure 2 comprises 2 culture chambers 4, one of the 2 culture chambers 4 being dedicated to provide a baseline measurement (i.e. control/reference 4b).

In another particular embodiment, each culture chamber 4 may have dimensions of 10 mm x 10 mm x 1.5 mm (e.g. L x W x H), more preferably comprised between 5 mm x 5 mm x 1 mm, still more preferably of about 4 mm x 4 mm x 0.5 mm.

In a particular embodiment of the present disclosure, said mixture 7 comprises at least one mixing enhancer 10 configured to be moved by a manipulating platform 11, preferably an external manipulating platform. The inventors have surprisingly observed that having at least one mixing enhancer 10 in said mixture 7 provides a more sensitive and rapid microfluidic extracellular environment monitoring device 1.

The mixing enhancement methods may be of active nature whereby the microfluidic mixing is achieved through an external force acting on the liquid (e.g said sample solution 6) inside the culture chamber 4, or a force acting on elements (e.g. magnetic elements 12) inside the culture chamber 4 manipulated by an external manipulating system 11. An example of the former, may be mixing induced by dispersing nano-sized particles into liquids i.e. ultrasonic or mega sonic, where the technique would require a transducer placed on in contact, or not, with the fluid (e.g. the sample solution 6) in the culture chamber 4. An example of the latter may be mixing induced by magnetic forces where magnetic elements 12 are placed inside the culture chamber 4 and they are manipulated externally with an (electro) magnet (array) (i.e. manipulating platform 11).

In a preferred embodiment of the present disclosure, said at least one mixing enhancer 10 comprises magnetic elements 12 configured to be moved by a magnetic field and being incorporated in at least one entity 13. For example, said magnetic field is induced by an electromagnet (array) (i.e. manipulating platform 11).

In a more preferred embodiment, said at least one entity 13 is a bead.

The inventors have surprisingly observed that having at least one mixing enhancer 10, preferably comprising magnetic elements 12 incorporated in at least one entity 13 in said mixture 7 provides a more sensitive and rapid microfluidic extracellular environmental monitoring device 1. For example, the application of a magnetic field may induce the movement of said at least one entity 13, preferably said at least one bead, incorporating said magnetic elements 12.
In still another preferred embodiment, said at least one luminescent chemical sensor 9 is in, on and/or about said at least one mixing enhancer 10.

In a particular embodiment, said at least one luminescent chemical sensor 9 is incorporated in said mixing enhancer 10, preferably in said entity 13 (Figures 1a and 1b; Figure 2, panels A, B and D).

In another particular embodiment of the present disclosure, said at least one mixing enhancer 10, preferably said at least one entity 13 incorporating said magnetic elements 13, may be positioned, preferably using a magnetic field induced by an electromagnet (array), for example on the top of said at least one culture chamber 4.

The term "top" within the meaning of the present disclosure corresponds to the top wall of said culture chamber 4 which is the wall opposite to the lower wall (bottom), the lower wall being the wall intended to rest on its support.

In some embodiments, said at least one mixing enhancer 10, preferably said at least one entity 13, incorporates both said at least one luminescent chemical sensor 9 and said magnetic elements 12 (Figures 1a and 1b; Figure 2, panels A, B and D). In such a manner, the mixing enhancer 10 may be localized in a specific location of the culture chamber 4, for example when conducting the readout, which will maximize the luminescent intensity and will therefore increase sensitivity of the microfluidic device 1.

In another preferred embodiment, said at least one luminescent chemical sensor 9 is about said at least one mixing enhancer 10, preferably about said at least one entity 13 incorporating said magnetic elements 12 (Figures 1c). In such a manner, the mixing enhancer 10 may be localized in a specific location of the culture chamber 4, for example when conducting the readout, which will reduce noise during readout and will therefore increase sensitivity of the microfluidic device.

In a particular embodiment of the present disclosure, said at least one entity 13, preferably said at least one bead, exhibits a magnetic behavior preferably superparamagnetic behavior.

Superparamagnetic matrices do not exhibit any magnetic behavior in the absence of a magnetic field, but once the magnetic field is present, the matrices magnetize and at specific magnetic element concentrations they chain up. If the magnetic elements are found in the presence of a rotating magnetic field then the chains move thereby mixing the environment around them. This mixing event contribute to provide a more sensitive and rapid microfluidic extracellular environment monitoring device.

Preferably, said magnetic elements 13 are ferromagnetic elements, more preferably, ferrite magnetic material-based nanoelements, neodymium, alnico or samarium cobalt, still more preferably iron oxide-based nanoelements. Said ferromagnetic elements may be stimulated in a controlled manner via the establishment of a magnetic field. In doing so, the ferromagnetic elements being incorporated in at least one matrix bead can be moved around the culture chamber.

In another particular embodiment of the present disclosure, said at least one entity 13, preferably said bead, is coated with at least one biological recognition molecule 14.

As used herein, the term "biological recognition molecule" refers to a biological molecule that can recognize and/or bind to a desired/specific analyte.

Preferably, said biological recognition molecule is an enzyme, an antibody, a protein, an oligonucleotide and/or DNA (Figure 2, panel B). According to this particular embodiment, the microfluidic device 1 of the present disclosure allows to detect but also to identify with specificity the analyte (e.g. a microbe) potentially present in the sample solution 6. In the case the analyte is a microbe, this is especially helpful when the urgent administration of a suitable antimicrobial agent is required to treat a potential fatal infection. In addition, coating said at least one entity 13 with at least one biological recognition molecule 14, for example an antibody, provides a microfluidic device 1 which is even more sensitive, since it allows to detect and/or identify very low numbers of analytes (e.g. microbe) in the sample 6.

In a preferred embodiment of the present disclosure, said at least one biological recognition molecule 14 is an antibody.

In a more preferred embodiment, said antibody is monoclonal, polyclonal or recombinant.

In a particular embodiment of the present disclosure, said antibody targets a surface antigen or a secreted toxin.

The biological recognition molecule 14 can be luminescent or bioluminescent. Preferably, said biological recognition molecule comprises a fluorescent labeling.

More preferably, said biological recognition molecule 14 is an antibody which is directly or indirectly linked to a fluorescent labeling.

In a particular embodiment of the present disclosure, said at least one mixing enhancer 10, preferably said at least one entity 13 incorporating said magnetic elements 12, is coated with said at least one biological recognition molecule 14, preferable an antibody, and incorporates said at least one luminescent chemical sensor 9.

In some embodiments, said extracellular environmental conditions are pH, O2, CO2, ammonia, calcium, magnesium, lactate, metals, cortisol, glucose, extracellular Adenosine Triphosphate (eATP) and/or polymers like glycans.

To sustain microbial growth, all microbes rely upon a range of metabolic processes from which they derive energy. By-products of these reactions are secreted into the extracellular environment.

The microfluidic device 1 according to the present disclosure and especially said at least one luminescent chemical sensor 9 is able to detect these changes in the microbial growth environment i.e. in the sample solution 6 if a microbe is present.

In a more preferred embodiment of the present disclosure, said extracellular environmental conditions are related to microbial growth, e.g. the pH, the O2 concentration and/or the CO2 concentration.

The growth of the microbe is determined by measuring e.g. the pH, the O2 concentration or the CO2 concentration. These environmental conditions are measured using said at least one luminescent chemical sensor 9 which exhibits altered emission spectra (either in terms of the emission wavelength or the emission intensity) when exposed to changes in the chemical composition of the environment (Figure 3). For example, pH has been shown to decrease for E. coli under aerobic conditions.

Advantageously, said microbe is selected in the group consisting of a virus, a fungi, a bacteria, a parasite, and their mixtures.

In a particularly advantageous embodiment, said microbe is a bacteria.

In a particular embodiment of the present disclosure, said at least one luminescent chemical sensor is a fluorescence-based dye.

Preferably, said at least one luminescent chemical sensor is selected from the group consisting of quenchable fluorophores, thiols, ruthenium(II), osmium(II), rhenium(I), iridium(III), platinum(II) and palladium(II), rhodamine based dyes, indicators for Ca2+, indicators for Na+, indicators for K+, indicators for Cl-, potential-sensitive dyes, luciferase-based sensors and their mixtures. Examples of quenchable fluorophores that may be used according to the present disclosure are SNARF, dichlorofluorescein derivatives, fluorescein derivatives.

Examples of thiols that may be used according to the present disclosure are BODIPY derivatives, alexa fluor malemides, naphthofluoresceins, chromoionophors, Styo 9 stain, resazurin, Lysosensor (blue, green, yellow/blue).

Examples of indicators for Ca2+ that may be used according the present disclosure are Aequorin, Fluo-3, Fluo-4, Rhod-2, Calcium GreenTM, Calcium OrangeTM, Calcium CrimsonTM, Oregon Green^{®} 488 BAPTA, Fura RedTM, Calcein.Examples of indicators for Na+ that may be used according the present disclosure are SBFI, Sodium Green, CoroNA Green. Example of indicators for K+ that may be used according to the present disclosure is PBFI. Examples of indicators for Cl- that may be used according to the present disclosure are 6-methoxyquinolinium derivatives, and Lucigenin. Examples of luciferase-based sensors that may be used according to the present disclosure are sensors based on the oxidation of D-luciferin by the luciferase enzyme.

In a preferred embodiment of the present disclosure, said at least one bead 13 is in the form of polymer beads, the polymer being preferably chosen in the group consisting of polystyrene, polyesters, silica, polytetrafluoroethene, poly (vinyl chlorides), poly (vinyl alcohols), plasticized PYC, polyurethanes, silicones, polyethylene, poly (tetrafIuorethylene) (PTFE), ethyl cellulose, polyacrylamides, poly(hydroxyethyl acrylate), poly (vinyl alcohols), poly(vinyl pyrrolidones), polyglycols, bio-based plastics, and their mixtures..

In a particularly advantageous embodiment of the present disclosure, said at least one sample solution 6 is a biological sample solution obtained from a body fluid, the body fluid being preferably selected from the group consisting of urine, blood, lymph, saliva, cerebrospinal fluid, milk and their mixtures.

In still another particular embodiment, said at least one sample solution 6 is a biological sample solution obtained from a body tissue, the body tissue being preferably selected from the group consisting of dental tissues, wax, connective tissues, such as cartilage, and their mixtures.

In still another particular embodiment, said at least one sample solution 6 is water and/or milk. In a particular embodiment, said at least one sample solution 6 is infected with a microbe.

Preferably, said microfluidic extracellular environment monitoring device 1 is a microfluidic bacterial growth testing device.

According to some embodiments, said sample solution 6 is exposed to said at least one luminescent chemical sensor 9 within said at least one culture chamber 4 wherein it will be determined whether or not a microbe is present in said sample solution 6. For this to occur, said mixture 7 partially coating said microstructure 2 and comprising said at least one luminescent chemical sensor 9 will dissolve upon loading of the sample solution 6 within said at least one culture chamber 4. If a microbe is present, said chemical sensor will sense change in extracellular environment conditions compared to a control/ reference. On the contrary, if said sample 6 is not infected, this in turn will result in minimal or no change to the chemical sensor 9 readout (i.e. emission spectrum) compared to a control/reference (Figure 3).

In some embodiments, said sample solution 6 is directly loaded into said at least one opening 5, without any preparation and/or pretreatment of said sample solution 6 before loading.

In a particular embodiment, said at least one biological sample solution 6 is obtained from said body fluid and/or said body tissue and is directly loaded intro said at least one opening, i.e. without any preparation and/or pretreatment of said body fluid and/or said body tissue before loading. For example, said at least one biological sample solution 6 is said body fluid, such as urine or blood.

In another particular embodiment, said at least one biological sample solution 6 is obtained by preparing and/or pretreating said body fluid and/or said body tissue before loading. For example, plasma can be separated from blood or urine can be purified. Also, to obtain said at least one biological sample solution 6 from a body tissue, said body tissue can be cut or crushed, and/or suspended in a buffer.

Advantageously, said microstructure 2 further comprises a secondary channel 15 with a first end 15a and a second end 15b, said at least one culture chamber 4 being opened to said first end 15a of said secondary channel 15 to enable a gas, preferably air, to exit the device 1 from said at least one culture chamber 4 and flow from said first end 15a to said second end 15b of said secondary channel 15, said secondary channel 15 further comprises a capillary stop 16 arranged between said first end 15a and second end 15b of said secondary channel 15 to prevent said at least one sample 6 to exit from said at least one culture chamber 4 (Figures 4 and 6).

In some particular embodiments, said secondary channel 15 further comprises an secondary opening 20 coupled to said second end 15b of said secondary channel 15 to enable a gas, preferably air, to exit the device 1 from said at least one culture chamber 4 and flow from said first end 15a to said second end 15b and said secondary opening 20 of said secondary channel 15.

In some embodiments, when said secondary opening 20 of said secondary channel 15 is present, said top layer 2c and said middle layer 2b are arranged to allow said outlet 19 and said secondary opening 20 to stack (be aligned) in such a manner that a gas, preferably air, is able to flow from said secondary opening 20 into said outlet 19.

In a particular embodiment of the present disclosure, said microstructure 2 comprises a plurality of culture chambers 4 (Figures 5 and 6), each culture chamber 4 being arranged in parallel, in series or circularly to enable said at least one sample solution 6 to flow from said first end 3a to said second end 3b of said primary channel 3 into said plurality of culture chambers 4.

In a more preferred embodiment, said microstructure 2 comprises a plurality of culture chambers 4, each culture chamber 4 being arranged in parallel, in series, or circularly to enable said at least one sample solution 6 to flow from said first end 3a to said second end 3b of said primary channel 3 into said plurality of culture chambers 4, said microstructure 2 further comprising a plurality of secondary channels 15 with a first end 15a and a second end 15b, each culture chamber 4 being opened to said first end 15a of said plurality of secondary channels 15 to enable a gas, preferably air, to exit the device 1 from each culture chamber 4 and flow from said first end 15a to said second end 15b of said plurality of secondary channels 15, each secondary channel 15 of said plurality of secondary channels 15 further comprising a capillary stop 16 arranged between said first end 15a and second end 15b of said plurality of secondary channels 15 to prevent said at least one sample 6 to exit from said plurality of culture chambers 4 (Figure 6).

In another particular embodiment of the present disclosure, said mixture 7 further comprises at least one predetermined drug 17, said at least one predetermined drug 17 being, preferably, an antimicrobial (Figures 1a-d).

In a preferred embodiment, the antimicrobial is selected in the group consisting of antibacterial (i.e. antibiotic), antiviral, antifungal, antiparasitic, and their mixtures. More preferably, the antimicrobial is an antibiotic.

In some embodiments, each culture chamber 4 of said plurality of culture chambers 4 may comprise same or different predetermined drugs 17, preferably antimicrobials, more preferably antibiotics, according to same or different concentrations. In a more preferred embodiment of the present disclosure, the microfluidic extracellular environment monitoring device 1 is an antimicrobial, preferably an antibacterial, susceptibility testing (AST) device.

According to certain embodiments of the present disclosure, said sample solution 6 containing a microbe is exposed to said at least one luminescent chemical sensor 9 within said at least one culture chamber 4 wherein it will be determined whether or not the microbe is susceptible to a specific antimicrobial 17 or not. For this to occur, said mixture 7 partially coating said microstructure 2further comprises the desired antimicrobial 17. Said mixture 7 partially coating said microstructure 2 and comprising said at least one luminescent chemical sensor 9 and said antimicrobial 17 will dissolved upon loading of the sample solution 6 within said at least one culture chamber 4. If the microbial continue to exhibit growth despite the presence of the antimicrobial 17 within said at least one culture chamber 17, the microbe will be regarded as being resistant to said antimicrobial 17 (thus the antimicrobial as being ineffective for this specific microbe). On the contrary, when an efficient antimicrobial 17 is tested, the microbe of interest will either cease growing or die. This in turn will result in minimal or no change to the chemical sensor 9 readout (i.e. emission spectrum) (Figure 3) compared to a control/reference (e.g. anon- infected sample).

Although the microfluidic extracellular environment monitoring device 1 shown herein may be used for bacterial growth measuring or for antimicrobial susceptibility testing, this is not by way of limitations of the embodiments of the present disclosure. The embodiments taught herein may also be used for other applications.

In a preferred embodiment, said at least one culture chamber 4 or each of said plurality of culture chambers 4 comprises a plurality of cavities (not represented), each cavity being arranged to enable said at least one sample solution 6 to flow from said first end 3a to said second end 3b of primary channel3 into said plurality of cavities, each cavity comprising a predetermined concentration of a same antimicrobial 17 so that a concentration gradient exists between each of the plurality of cavities.

Another object of the present disclosure is an extracellular environment monitoring system comprising:
- the microfluidic device 1 according to the present disclosure;
- at least one electromagnet 11 arranged to provide a magnetic flux through said at least one culture chamber 4; and
- a readout device (not represented) for monitoring data relative to extracellular environmental conditions in said at least one culture chamber 4, said data corresponding to said emission spectrum of said at least one chemical sensor 9.

In a particular embodiment of the present disclosure, said data relative to
extracellular environmental conditions are data relative to microbial growth.

Preferably, said system may also comprise an imaging system (not represented), preferably a high power microscope, arranged to image microbial cells in said at least one culture chamber 4. More preferably, said imaging system monitors data relative to microbial growth.

Preferably, monitoring the growth of the microbe comprises S/I/R determinations about the antimicrobial and the microbe and/or a minimal inhibitory concentration (MIC) determination of the antimicrobial.

Still more preferably, monitoring the growth of the microbe comprises using said imaging system to count the average number of microbe per culture chamber.

All the preferred and particular embodiments of the microfluidic extracellular environment monitoring device 1 according to the present disclosure apply identically to the extracellular environment monitoring system.

Another object of the present disclosure is a method for extracellular environment monitoring, the method comprising:
- obtaining a microfluidic extracellular environment monitoring device 1 according to the present disclosure;
- loading a sample solution 6 into said microstructure 2 through said at least one opening 5 coupled to said first end 3a of said primary channel 3, to flow along said primary channel 3 from said first end 3a to said second end 3b into said at least one culture chamber 4, allowing said sample solution 6 to contact with said coated mixture 7 in said microstructure 2; and
- monitoring data with a readout device relative to extracellular environmental conditions in said at least one culture chamber 4, said data corresponding to said emission spectrum of said at least one chemical sensor 9.

Within the present disclosure, said sample solution 6 is exposed to said at least one luminescent chemical sensor 9 within said at least one culture chamber 4.

According to a preferred embodiment, within the culture chamber 4, it is determined if a microbe is present or not. For this to occur, said at least one culture chamber 4 comprises a mixture 7 coating at least partially said microstructure 2 and comprising at least one luminescent chemical sensor 9, said mixture 7 being dissolved once the sample solution 6 is loaded into said primary channel 3 and into said at least one culture chamber 4 open to said primary channel 3 and enters into contact with said mixture 7 deposited as a coating.

Depending on the luminescent chemical sensor 9, one or more extracellular environmental conditions can be measured, for example O2 concentration CO2 concentration and/or pH, which alter the luminescent behavior of the sensor 9. The chemical sensor 9 can be excited at a specific wavelength (i.e. excitation wavelength) based on the excitation spectrum of the chemical sensor 9. The preferred behavior of the chemical sensor 9 is a change in intensity of the emission spectrum, which directly correlates to changes in the environment (i.e. changes in the pH, O2 concentration, and/or CO2 concentration) (Figures 2 and 3).

In a particular embodiment of the present disclosure, said mixture 7 comprises at least one mixing enhancer 10 configured to be moved by a magnetic field.

When said mixture 7 further comprises said at least one mixing enhancer 10, preferably magnetic elements (12) configured to be moved by a magnetic field and being incorporated in at least one entity (13), preferably the entity (13) being a bead, the method according to the present disclosure further comprises:
- applying a magnetic field within said at least one culture chamber 4 to move said magnetic elements 12.

Said magnetic field may comprise a magnetic flux having a determined orientation which can, preferably, be switched, for witching the magnetic flux orientation. Said magnetic flux may be switched on or off. The switching of magnetic flux orientation or the switching on and off of the magnetic flux is to initiate mixing of the mixture dissolved and the sample solution 6. In addition, the manipulation of the magnetic flux, can be used to position the magnetic elements 12 at specific locations within the device 1 to optimize or improve the readout.

The inventors have surprisingly observed that having at least one mixing enhancer 10 in said mixture 7 provides a more sensitive and rapid microfluidic extracellular environment monitoring device 1. Preferably, said magnets may be electromagnets.

Advantageously, said method further comprises, by applying said magnetic field:
- inducing the movement of said at least one mixing enhancer 10, said movement having preferably a superparamagnetic behavior.

In a particular embodiment of the method of the present disclosure, said method further comprises, by applying said magnetic field:
- localizing said at least one mixing enhancer 10 in a specific location of the culture chamber 4.

In a particular embodiment of the present disclosure, the method further comprises:
- assessing the presence of a microbe, said emission spectrum, when said microbe is contacted with said mixture 7, being compared with a baseline measurement.

In still another particular embodiment of the present disclosure, the method further comprises:
- assessing the efficiency of said at least one predetermined drug 17, said emission spectrum, when said microbe is contacted with said mixture 7 comprising said at least one predetermined drug 17, being compared with a baseline measurement.

All the preferred and particular embodiments of the microfluidic extracellular environment monitoring device according to the present disclosure apply identically to the method for extracellular environment monitoring.

Another object of the disclosure is a use of the microfluidic extracellular environment monitoring device 1 for detecting a microbe present in a sample solution 6.

All the preferred and particular embodiments of the microfluidic extracellular environment monitoring device 1 according to the present disclosure apply identically to the use of the microfluidic extracellular environment monitoring device for detecting a microbe present in a sample solution 6.

Still another object of the disclosure is a use of the microfluidic extracellular environment monitoring device 1 for detecting the susceptibility of a microbe present in a sample solution 6 to a predetermined drug 17.

All the preferred and particular embodiments of the microfluidic extracellular environment monitoring device 1 according to the present disclosure apply identically to the use of the microfluidic extracellular environment monitoring device 1 for detecting the susceptibility of a microbe present in a sample solution 6 to a predetermined drug 6.

These and other embodiments of the disclosure are indicated in the appended EEs.

The disclosure will now be further described with reference to the following examples, which show non-limiting embodiments of different aspects of the disclosure.

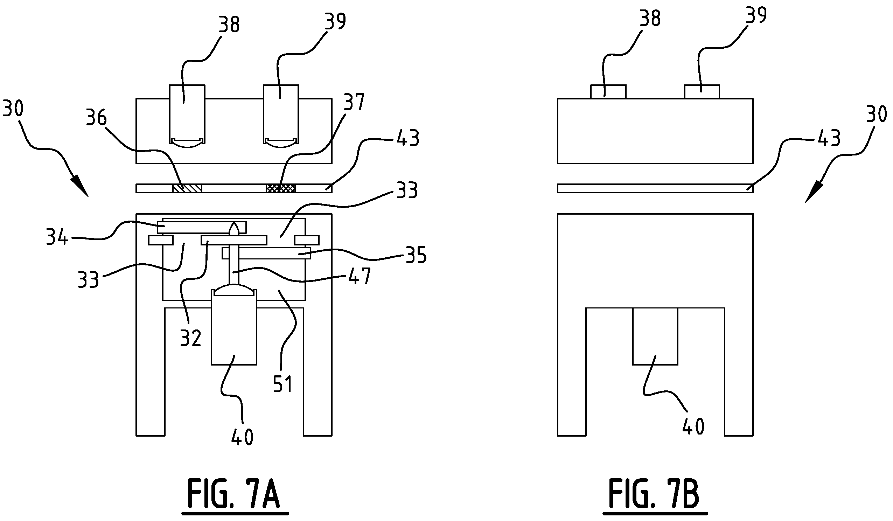

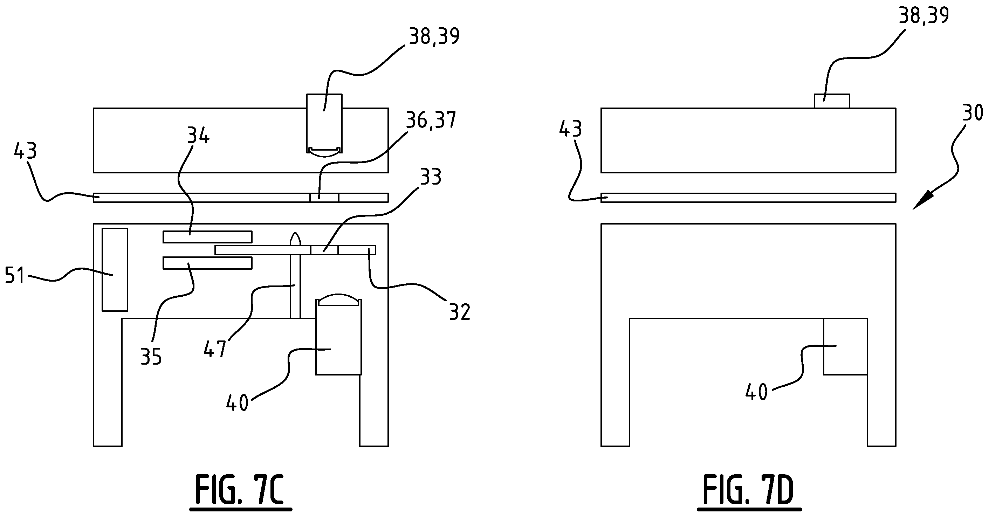

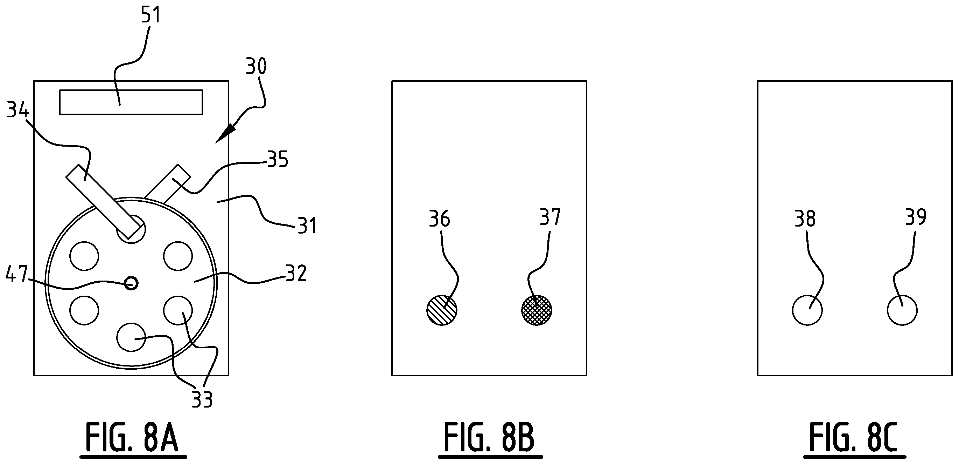

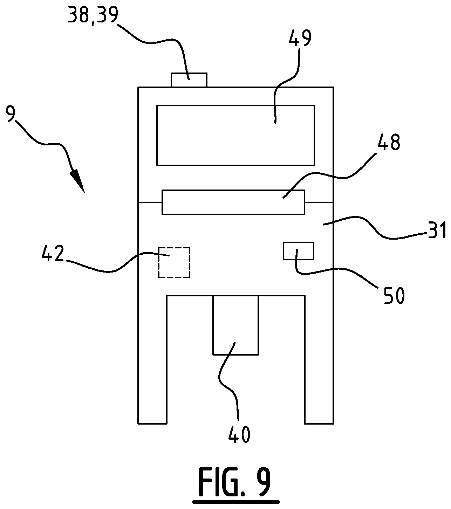

Figures 7-9 show an embodiment of a detecting device according to an embodiment of the present disclosure. Figure 9 is an overall view of the detecting device. Figures 7A-7D are a cut-away front view, a front view, a cut-away side view and a side view, respectively, of the embodiment of figure 9. Figures 8A-8C respectively show the lower half of the detecting device in top view, the mid part of the device in top view and the upper half of the detecting device, in top view as well.

A microfluidic extracellular environment monitoring system may comprise a microfluidic device 1, a detecting device 30 and/or a manipulating platform 11 (in some embodiments as part of the detecting device 30 but external to the monitoring device (1), see for instance the magnets 34, 35 that may be used to generate a magnetic field causing magnetic particles in the sample chamber to move thereby entraining at least some of the other components of the mixture inside the sample chamber. The detecting device 30 may be comprised of a housing 31 provided with a holder 32 for holding the at least one microfluidic extracellular environment monitoring device 1, a light source 40 configured to direct a light beam to the sample chamber, a support plate 43 provided with one or more optical filter 36, 37 arranged to pass only light with a predetermined wavelength (range) (for instance, green and red light), one or more light detectors 38, 39 configured to detect light emitted from the sample chamber, and a spectrum analyzer unit 42 (schematically shown in figure 9) configured to determine data representative of the varying emission spectrum of the detected emitted light resulting from the varying extracellular environmental conditions inside the sample chamber.

Furthermore, the housing 31 may comprise one or more displays 49, a slot 48, and on/off buttons 50. The display 49 may be used for displaying settings values and/or monitoring data values. The slot 48 may be configured to allow a user to insert a microfluidic device 1 into the detecting device (i.e. the readout device) 30 for monitoring the content of one or more sample chambers 4 for analysis. The slot 48 may further be configured to allow a user to remove the microfluidic device 1. The device 30 may further comprise input buttons for inputting monitoring settings of the readout device 1. A readout device may further comprise one or more input data ports, one or more output data ports and/or one or more combined input/output ports for data and/or settings of the detecting device 30.

The holder 32 comprises a number of holder openings 33. The positions of the holder openings 33 correspond to the positions of the sample chambers of the substrate of the monitoring device 1 placed on top of the holder. The holder openings 33 allow for the passage of one or more light beams from the light source 40, through the holder openings 33, the one or more sample chambers and through the optical filter 36, 37. Furthermore, the holder 32 is rotatably mounted to the housing using a rotation shaft 47 connected to a drive motor (not shown). The rotation shaft 47 is configured to intermittently rotate the holder 32 and the monitoring device 1 (for instance comprising a substrate as shown in figure 5 or 6) placed on top of the holder 32 so that the plurality of sample chambers 4 of an inserted substrate may consecutively be positioned in the light beam originating from the light source 40. The one or more light detectors 38, 39 are configured to receive the light that is emitted in turn by the sample solution (6) and then filtered by the one or more optical filters 36, 37. The filtered light is then detected by the light detectors. More specifically, light arriving from the first optical filter 36 (for instance, red light) is collected by the first light sensor 38 and light arriving from the second optical filter 37 (for instance, green light) is collected by the second light sensor 37. Since the light sources are each arranged in a separate light chamber, light from the first optical filter 36 will only reach the first light detector 38 and light from the second optical filter 37 will only reach the second light detector 39. By consecutively rotating the holder the same sample chamber is measured twice (once by each of the light detectors). The shown embodiment has the advantage that the light detectors can say at fixed positions and no further actuators and/or rotating parts are needed, which makes the detecting device relatively simple and reliable.

As mentioned above, the light received in both light chambers is measured in terms of emissions, and then correlated afterwards for which chamber each emission wavelength corresponds to. Furthermore, the light detectors 38, 39 may form a ratiometric sensor configured to provide a sensor output representative of the ratio of the detected light of the first wavelength (for instance, red light) and the detected light of the second wavelength (for instance, the green light) so that the sensor output = red/green. This ratiometry is used as a method to reduce dependencies and heterogeneities arising from initial sensor concentrations or depositions.

In embodiments of the detecting device the device is embodied as an incubator as well. For instance, the detecting device may be configured to allow the substrate and its contents (i.e. the sample solution) to be maintained under controlled environmental conditions, for instance in terms of temperature and/or humidity. To this end one or more heaters 51 may be present inside the housing 31. Alternatively or additionally, a humidity control element (not shown in the figures) may be arranged inside the housing as well. In some applications the heater may control the temperature to be maintained within a temperature range of 30-40 C, preferably in the range of 35-37C. This is the temperature range within which microorganisms, such as bacteria, usually are allowed to grow.

### EXAMPLES

### Manufacturing process of the microfluidic device according to some embodiments of the present disclosure

The middle layer 2b is first attached to the bottom layer 2a and pressure is applied to ensure adhesion.

2 µL of pluronic acid F-127 (1% w v-1) solution is then deposited in all the cavities (e.g. said at least one opening 5, said primary channel 3 and/or said at least one culture chamber 4) in the middle layer 2b and left to dry at room temperature overnight. This step hydrophilizes the PMMA surface to promote fluid distribution in said at least one culture chamber 4 and remove present air bubbles.

Next, 1 µl of mixture 7 of 5-25% glycerol, SNARF pH sensor, LB (lysogeny broth) nutrients, and 6000 magnetic beads/ µl (and optionally antibiotics) is deposited as a drop in the middle of said culture chamber 4, resulting to a partial coating of the microfluidic detection chamber. The resulting arrangement resembles a liquid drop confinement in the center of the chamber.

Finally, the top layer 3c is aligned and attached to the middle layer 2b. Pressure is applied.

### Example of a method for extracellular environment monitoring according to the present disclosure

A sample of urine 6 infected with a microbe is loaded into said microstructure 2 through said at least one opening 5 couple to said first end 3a of said primary channel 3 and flow along said primary channel 3 into 2 culture chambers 4. The sample of urine then enters in contact with said mixture 7 being deposited as a coating having the form of a drop in said 2 culture chambers 4 and dissolves said mixture 7. The first culture chamber 4 corresponds to the control/reference (no antibiotic). Different antibiotics are tested, each being present in said mixture 7 in a distinct culture chamber 4.

Then a magnetic field is applied within culture chambers 2 to move said magnetic beads 12 present in said mixture 7.

The emission spectrum of the pH sensor is monitored at specific times (e.g. 1 hour, 2 hours, 3 hours, 4 hours, 6 hours, 8 hours from sample loading) with a readout device.

The susceptibility of the microbe 18 present in the sample of urine 6 to the different tested antibiotics is detected. In the case of an ineffective antibiotic, bacterial growth will ensue, accompanied by pH changes as well as an increase in the number of bacteria, whereas in the presence of effective antibiotics, bacterial growth will be hindered or will not occur.

The disclosure further relates to the following numbered exemplifying embodiments (EE's).

EE 1. Microfluidic extracellular environment monitoring device (1) comprising:
- a substrate comprising a microstructure (2) formed therein, the microstructure (2) comprising:
- a channel (3) with a first end (3a) and a second end (3b), and
- a sample chamber (4) that opens to the channel (3); and
- an opening (5) in fluid communication with the microstructure (2) via the first end (3a) of the channel (3);

wherein the opening (5) is configured to load at least one sample solution (6) into the microstructure (2) and the microstructure (2) is configured to induce a flow of the at least one sample solution (6) from the first end (3a) to the second end (3b) of the channel (3) into the sample chamber (4);
wherein at least one of the channel (3) and the sample chamber (4) comprises a part which is coated with a mixture (7), the mixture (7) comprising at least the following components:
   - a matrix element (8);
   - at least one luminescent chemical sensor material (9) configured to emit an emission spectrum dependent on extracellular environmental conditions in the microstructure (2);
wherein each of the components of the mixture (7) is selected to be at least partially dissolvable or dispersible in the at least one sample solution (6) so as to mix or diffuse with the sample solution (6).

EE 2. Microfluidic extracellular environment monitoring device (1) according to EE 1, wherein the components of the mixture (7) are selected such that the mixture (7) is at least 40% or 50%, preferably at least 70%, more preferably at least 90% dissolvable or dispersible in the at least one sample solution (6).

EE 3. Microfluidic extracellular environment monitoring device (1) according to EE 1 or 2, wherein the mixture (7) is configured to be substantially entirely or almost diffused to the sample solution (6) within 10 minutes, preferably within 5 minutes, even more preferably within 1 minute, after loading of the at least one sample solution into the microstructure (2).

EE 4. Microfluidic extracellular environment monitoring device (1) according to EE 1, 2 or 3, wherein the components of the coated mixture (7) are selected to be dissolvable or dispersible in at least one sample solution (6) comprising a water-based substance.

EE 5. Microfluidic extracellular environment monitoring device (1) according to EE 4, wherein the water-based substance is a body fluid or fluid derived from a body fluid, the body fluid optionally being selected from the group consisting of urine, blood, lymph, saliva, wax, cerebrospinal fluid, milk and their mixtures, and/or the body fluid being obtained from a body tissue, the body tissue optionally being selected from the group consisting of dental tissues, connective tissues, such as cartilage, and their mixtures.

EE 6. Microfluidic extracellular environment monitoring device (1) as defined in any of the preceding EE's, wherein a further component of the mixture (7) is a microbial growth agent, preferably the microbial growth agent being a bacterial nutrient, more preferably lysogeny broth or Mueller Hinton Broth and/or polysaccharides, wherein the microbial growth agent preferably is at least partially or fully dissolvable or dispersible in the sample solution.

E 7. Microfluidic extracellular environment monitoring device (1) as defined in any of the preceding EE's, wherein a further component of the mixture (7) is a predetermined drug (17), preferably the at least one predetermined drug (17) being an antimicrobial, more preferably an anti-bacterial, still more preferably an antibiotic, wherein the predetermined drug (17) preferably is least partially or fully dissolvable or dispersible in the sample solution.

EE 8. Microfluidic extracellular environment monitoring device (1) according to any one of the preceding EE's, wherein a further component of the mixture is a mixing enhancer (10) configured to enhance mixing of a dissolved or dispersed component by cooperation with a fully external manipulating platform (11).

EE 9. Microfluidic extracellular environment monitoring device (1) according to EE 8, wherein the mixing enhancer (10) comprises at least one entity (13) which incorporates magnetic elements (12), the magnetic elements being configured to be moved by a magnetic field generated by the fully external manipulating platform (11), wherein preferably the at least one entity (13) is a bead and wherein preferably the bead has a wall defining an internal volume wherein the magnetic elements are arranged.

EE 10. Microfluidic extracellular environment monitoring device (1) according to EE 9, wherein the magnetic elements (12) are ferromagnetic elements, preferably ferrite magnetic material-based nanoelements, neodymium, alnico or samarium cobalt, more preferably iron oxide-based nanoelements.

EE 11. Microfluidic extracellular environment monitoring device (1) according to EE 9 or 10, wherein the at least one entity (13) is coated with at least one biological recognition molecule (14), for example an antibody.

EE 12. Microfluidic extracellular environment monitoring device (1) according to any one of the preceding EE's, wherein the at least one luminescent chemical sensor material (9) is selected to move freely relative to microbes (18) present in the sample solution (6) when dissolved or dispersed in the sample solution (6).

EE 13. Microfluidic extracellular environment monitoring device (1) according to any one of the preceding EE's, wherein the at least one luminescent chemical sensor material (9) is arranged inside an entity (13) and/or at a wall of an entity (13), so as to not directly contact microbes (18) present in the sample solution (6) when dissolved or dispersed in the sample solution (6).

EE 14. Microfluidic extracellular environment monitoring device (1) according to any one of the preceding EE's, wherein the at least one luminescent chemical sensor material (9) is selected to not penetrate microbes (18) present in the sample solution (6).

EE 15. Microfluidic extracellular environment monitoring device (1) as defined in any of the preceding EE's, comprising a plurality of the channels (3) and associated sample chambers (4), each channel having a first end (3a) in fluid connection with an opening for loading the at least one sample solution (6) and a second end (3b) in fluid connection with a sample chamber (4), wherein openings of the plurality of channels (3) preferably form a single common opening for leading the at least one sample solution (6) into each of the channels (3).

EE 16. Microfluidic extracellular environment monitoring device (1) as defined in EE 14, wherein coated parts of each of the channels and sample chambers are coated by a mixture (7) comprising a different predetermined drug (17).

EE 17. Microfluidic extracellular environment monitoring device (1) as defined in any of the preceding EE's, wherein the mixture (7) comprises a plurality of different mixtures, the mixtures comprising different components and/or different concentrations of components.

EE 18. Microfluidic extracellular environment monitoring device (1) as defined in any of the preceding EE's, wherein at least one of the channel (3) and sample chamber (4) has a first part coated with a first mixture and second part coated with a second mixture, different from the first mixture.

EE 19. Microfluidic extracellular environment monitoring device (1) as defined in EE 18, wherein only the first mixture comprises the chemical sensor material and/or only the second mixture comprises a microbial growth agent and/or a predetermined drug.

EE 20. Microfluidic extracellular environment monitoring device (1) according to any of the preceding EE's, wherein the sample chamber (4) is at least partially transparent to the outside of the microstructure (2), wherein preferably substantially the entire substrate is transparent.

EE 21. Microfluidic extracellular environment monitoring device (1) as defined in any of the preceding EE's, wherein the coated part is present only in the channel (3).

EE 22. Microfluidic extracellular environment monitoring device (1) as defined in any of the preceding EE's, wherein the coated part comprises a plurality of droplets of the mixture (7).

EE 23. Microfluidic extracellular environment monitoring device (1) as defined in any of the preceding EE's, wherein the coated part comprises a layer of the mixture (7).

EE 24. Microfluidic extracellular environment monitoring device (1) according to any one of the preceding EE's, wherein the coated part comprises a powder, liquid, semi-liquid, semi-solid, and/or gel.

EE 25. Microfluidic extracellular environment monitoring device (1) according to any one of the preceding EE's, wherein the mixture (7) has a viscosity greater than water, preferably greater than or equal to 0.85 cP, more preferably greater than or equal to 1 cP, still more preferably greater than or equal to 1.3 cP, yet still more preferably greater than or equal to 1.8 cP, at a temperature of 30 °C as measured using a viscometer.

EE 26. Microfluidic extracellular environment monitoring device (1) according to any one of the preceding EE's, wherein the luminescent sensing material (9) is configured to emit an emission spectrum dependent on extracellular environmental conditions including at least one of pH and the concentration of O₂, CO₂, ammonia, calcium, magnesium, metals, lactate, cortisol, glucose, extracellular Adenosine Triphosphate (eATP) and/or polymers like glycans.

EE 27. Microfluidic extracellular environment monitoring device (1) according to any one of the preceding EE's, wherein the at least one luminescent chemical sensor material (9) is selected from the group consisting of quenchable fluorophores, thiols, ruthenium(II), osmium(II), rhenium(I), iridium(III), platinum(II) and palladium(II), rhodamine based dyes, indicators for Ca²⁺, indicators for Na⁺, indicators for K⁺, indicators for Cl⁻, potential-sensitive dyes, luciferase-based sensors and their mixtures.

EE 28. Microfluidic extracellular environment monitoring device (1) according to any one of the preceding EE's, wherein the microstructure (2) further comprises:
a secondary channel (15) with a first end (15a) and a second end (15b), the at least one sample chamber (4) being opened to the first end (15a) of the secondary channel (15) to enable a gas, preferably air, to exit the microfluidic extracellular environment monitoring device (1) from the sample chamber (4) and flow from the first end (15a) to the second end (15b) of the secondary channel (15),
the secondary channel (15) optionally comprising a capillary stop (16) arranged between the first end (15a) and second end (15b) of the secondary channel (15) to prevent the at least one sample solution (6) from exiting the at least one sample chamber (4).

EE 29. Microfluidic extracellular environment monitoring device (1) according to any one of the preceding EE's, wherein the microstructure (2) comprises a plurality of the substructures, each substructure comprising a channel (3) with a first end (3a) and a second end (3b), and a sample chamber (4) in fluid communication with the channel (3), wherein the substructures are arranged in parallel, in series, or circularly, in the substrate to enable the at least one sample solution (6) to flow into respective substructures along the associated channels (3) from the first end (3a) directly to the second end (3b) of each channel (3) into each of the plurality of sample chambers (4).

EE 30. Microfluidic extracellular environment monitoring device (1) according to EE 29, wherein the sample chambers (4) of the plurality of substructures are arranged on one or more concentric imaginary circles with respect to the opening (5).

EE 31. Microfluidic extracellular environment monitoring device (1) according to any one of the preceding EE's, wherein the opening (5) comprises a filter, preferably a filter membrane, to filter unwanted substances from the sample solution (6).

EE 32. Microfluidic extracellular environment monitoring device (1) according to any one of the preceding EE's, wherein the microstructure (2) is configured to induce a capillary flow in the one or more channels.

EE 33. System for extracellular environment monitoring, the system comprising:
- at least one microfluidic extracellular environment monitoring device as defined in any of the preceding EE's;
- a detecting device comprising a holder for holding the at least one microfluidic extracellular environment monitoring device, a light source configured to direct a light beam to the sample chamber, a light detector configured to detect light emitted from the sample chamber, and a spectrum analyzer unit configured to determine data representative of the varying emission spectrum of the detected emitted light resulting from the varying extracellular environmental conditions inside the sample chamber.

EE 34. System as defined in EE 33, further comprising a manipulating platform (11) fully arranged outside the microfluidic extracellular environment monitoring device.

EE 35. Method for extracellular environment monitoring using a device (1) according to any of EE's 1-32 or a system as defined in EE 32 or 33, the method comprising:
- loading a sample solution (6) into a microstructure (2) through least one opening (5);
- inducing a flow of the loaded sample solution (6) from the at least one opening (5) along at least one channel to at least one sample chamber (4);
- allowing the sample solution (6) to contact with the mixture (7) so as to at least partially dissolve the mixture (7) in the sample solution (6);
- detecting light emitted from the sample chamber (4); and
- determining from the detected light a varying emission spectrum of the emitted light resulting from the varying extracellular environmental conditions inside the sample chamber.

EE 36. Method as defined in EE 35, wherein detecting light emitted from the sample chamber (4) comprises:
- directing a light beam from a light source to the sample solution (6) inside the sample chamber (4);
- detecting light emitted from the sample chamber (4), the emitted light being caused by luminescence in the sample solution inside the sample chamber.

EE 37. The method according to EE 35 or 36, further comprising, before allowing the sample solution (6) to contact with the mixture (7):
monitoring extracellular environmental conditions in the at least one sample chamber (4), for collecting data corresponding to the emission spectrum of the at least one chemical sensor material (9).

EE 38. The method according to any of EE's 35-37, comprising periodically or substantially continuously monitoring extracellular environmental conditions in the at least one sample chamber (4), in real-time during the allowing the sample solution (6) to contact with the mixture (7).

EE 39. The method according to any of the EE's 35-38, wherein a capillary flow occurs during 0.1-10 seconds and/or and/or wherein the monitoring is performed during 1-8 hours, preferably 3-5 hours.

EE 40. The method according to any of the EE's 35-39, wherein at least partially dissolving the mixture (7) comprises dissolving at least 40% or 50%, preferably at least 70%, more preferably at least 90% of the mixture (7), and most preferably comprises dissolving substantially all of the mixture (7).

EE 41. The method according to any of the EE's 35-40, comprising allowing mixture (6) to substantially dissolve or diffuse into the sample solution (6) within 10 minutes, preferably within 5 minutes, even more preferably within 1 minute, after loading of the at least one sample solution into the microstructure (2).

EE 42. The method according to any of the EE's 35-41, further comprising enhancing the mixing of the sample solution (6) with the mixture (7) by applying a magnetic field within the at least one sample chamber (4) to move a magnetic elements (12) present in the matrix (7).

EE 43. The method according to any of the EE's 35-42, further comprising enhancing the mixing of the sample solution (6) with the mixture (7) by applying ultrasound to the sample chamber (4).

EE 44. Use of a microfluidic device (1) according to any of EE's 1-35 for detecting a microbe (18) present in a sample solution (6).

EE 45. Use of a microfluidic extracellular environment monitoring device according to any one of the EE's 1-35 for detecting the susceptibility of a microbe (18) present in a sample solution (6) to a predetermined drug (17) and/or for detecting the relative susceptibility of a microbe (18) present in a sample solution (6) to a plurality of different predetermined drugs (17).

## Claims

1. Microfluidic device (1) for detecting the susceptibility of a microbe present in a sample solution (6), the device comprising:
- a substrate comprising a microstructure (2) formed therein, the microstructure (2) comprising a plurality of channels (3);
- wherein each channel (3) and /or each sample chamber (4) is coated with a mixture (7), the mixture (7) comprising:
- a luminescent chemical sensor l (9) dissolvable or dispersible in the sample solution (6) and configured to emit an emission spectrum dependent on extracellular environmental conditions in the microstructure (2)
wherein the mixture in at least one of the channels or chambers further comprises at least one predetermined drug (17), the at least one predetermined drug being an antimicrobial.

2. Microfluidic device (1) as claimed in claim 1, wherein the microfluidic device 1 is an antimicrobial susceptibility testing (AST) device, wherein the at least one predetermined drug (17) preferably is anti-bacterial, more preferably an antibiotic, wherein the predetermined drug (17) preferably is at least partially or fully dissolvable or dispersible in the sample solution.

3. Microfluidic device (1) as claimed in any of the preceding claims, wherein respective channels and sample chambers are coated by a mixtures (7) comprising a different predetermined drug (17) and/or wherein the mixture (7) comprises a plurality of different mixtures, the mixtures comprising different components and/or different concentrations of components.

4. Microfluidic device (1) as claimed in any of the preceding claims, wherein each channel (3) has a first end (3a) and a second end (3b), wherein the first end (3a) is in fluid communication with an opening (5) configured to load at least one sample solution (6) and wherein the second end (3b) is in fluid connection with the sample chamber (4), wherein openings (5) of the plurality of channels (3) preferably form a single common opening for leading the at least one sample solution into each of the channels (3) and/or wherein the microstructure (2) is configured to induce a flow of the at least one sample solution (6) from the first end (3a) to the second end (3b) of each of the channels (3) into an associated sample chamber (4), wherein the flow preferably is a capillary flow.

5. Microfluidic device (1) as claimed in any of the preceding claims, wherein the mixture (7) further comprises a matrix element (8), wherein preferably the luminescent chemical sensor and the matrix are selected to be at least partially dissolvable or dispersible in the at least one sample solution (6) so as to mix or diffuse with the sample solution (6).7. Microfluidic device (1) according to any of the preceding claims, wherein the mixture (7) is at least 40% or 50%, preferably at least 70%, more preferably at least 90% dissolvable or dispersible in the at least one sample solution (6) and/or wherein the mixture (7) is configured to be substantially entirely or almost diffused to the sample solution (6) within 10 minutes, preferably within 5 minutes, even more preferably within 1 minute, after loading of the at least one sample solution into the microstructure (2) and/or wherein the components of the coated mixture (7) are selected to be dissolvable or dispersible in at least one sample solution (6) comprising a water-based substance, wherein the water-based substance preferably is a body fluid or fluid derived from a body fluid, the body fluid optionally being selected from the group consisting of urine, blood, lymph, saliva, wax, cerebrospinal fluid, milk and their mixtures, and/or the body fluid being obtained from a body tissue, the body tissue optionally being selected from the group consisting of dental tissues, connective tissues, such as cartilage, and their mixtures.

6. Microfluidic device (1) as claimed in any of the preceding claims, wherein the mixture (7) comprises a microbial growth agent, preferably the microbial growth agent being a bacterial nutrient, more preferably lysogeny broth or Mueller Hinton Broth and/or polysaccharides, wherein the microbial growth agent preferably is at least partially or fully dissolvable or dispersible in the sample solution.

7. Microfluidic device (1) according to any one of the preceding claims, wherein a further component of the mixture is a mixing enhancer (10) configured to enhance mixing of a dissolved or dispersed component by cooperation with a fully external manipulating platform (11), wherein the mixing enhancer (10) optionally comprises at least one entity (13) which incorporates magnetic elements (12), the magnetic elements being configured to be moved by a magnetic field generated by the fully external manipulating platform (11), wherein preferably the at least one entity (13) is a bead and wherein preferably the bead has a wall defining an internal volume wherein the magnetic elements are arranged, wherein the magnetic elements (12) optionally are ferromagnetic elements, preferably ferrite magnetic material-based nanoelements, neodymium, alnico or samarium cobalt, more preferably iron oxide-based nanoelements and/or wherein the at least one entity (13) is coated with at least one biological recognition molecule (14), for example an antibody.

8. Microfluidic device (1) according to any one of the preceding claims, wherein the at least one luminescent chemical sensor (9) is selected to move freely relative to microbes (18) present in the sample solution (6) when dissolved or dispersed in the sample solution (6) and/or wherein the at least one luminescent chemical sensor (9) is arranged inside an entity (13) and/or at a wall of an entity (13), so as to not directly contact microbes (18) present in the sample solution (6) when dissolved or dispersed in the sample solution (6) and/or wherein the at least one luminescent chemical sensor (9) is selected to not penetrate microbes (18) present in the sample solution (6).

9. Microfluidic device (1) as claimed in any of the preceding claims, wherein the mixture (7) comprises a plurality of different mixtures, the mixtures comprising different components and/or different concentrations of components.

10. Microfluidic device (1) as claimed in any of the preceding claims, wherein at least one of the channel (3) and sample chamber (4) has a first part coated with a first mixture and second part coated with a second mixture, different from the first mixture, wherein preferably only the first mixture comprises the chemical sensor and/or only the second mixture comprises a microbial growth agent and/or a predetermined drug.

11. Microfluidic device (1) according to any one of the preceding claims, wherein the mixture (7) has a viscosity greater than water, preferably greater than or equal to 0.85 cP, more preferably greater than or equal to 1 cP, still more preferably greater than or equal to 1.3 cP, yet still more preferably greater than or equal to 1.8 cP, at a temperature of 30 °C as measured using a viscometer and/or wherein the luminescent sensor (9) is configured to emit an emission spectrum dependent on extracellular environmental conditions including at least one of pH and the concentration of O₂, CO₂, ammonia, calcium, magnesium, metals, lactate, cortisol, glucose, extracellular Adenosine Triphosphate (eATP) and/or polymers like glycans and/or wherein the at least one luminescent chemical sensor (9) is selected from the group consisting of quenchable fluorophores, thiols, ruthenium(II), osmium(II), rhenium(I), iridium(III), platinum(II) and palladium(II), rhodamine based dyes, indicators for Ca2+, indicators for Na+, indicators for K+, indicators for Cl-, potential-sensitive dyes, luciferase-based sensors and their mixtures.

12. Microfluidic device (1) according to any one of the preceding claims, wherein the microstructure (2) comprises a plurality of the substructures, each substructure comprising a channel (3) with a first end (3a) and a second end (3b), and a sample chamber (4) in fluid communication with the channel (3), wherein the substructures are arranged in parallel, in series, or circularly, in the substrate to enable the at least one sample solution (6) to flow into respective substructures along the associated channels (3) from the first end (3a) directly to the second end (3b) of each channel (3) into each of the plurality of sample chambers (4), wherein the sample chambers (4) of the plurality of substructures are preferably arranged on one or more concentric imaginary circles with respect to the opening (5).

13. Antimicrobial susceptibility testing (AST) system for detecting the susceptibility of a microbe present in a sample solution (6), the system comprising:
- at least one microfluidic device as claimed in any of the preceding claims;
- a detecting device comprising a holder for holding the at least one microfluidic device, a light source configured to direct a light beam to the sample chamber, a light detector configured to detect light emitted from the sample chamber, and a spectrum analyzer unit configured to determine data representative of the varying emission spectrum of the detected emitted light resulting from the varying extracellular environmental conditions inside the sample chamber, optionally comprising a manipulating platform (11) fully arranged outside the microfluidic extracellular environment monitoring device.

14. Method for extracellular environment monitoring using a microfluidic device (1) according to any of claims 1-12 or a system as claimed in claim 13, the method comprising:
- loading a sample solution (6) into a microstructure (2) through least one opening (5);
- inducing a flow of the loaded sample solution (6) from the at least one opening (5) along at least one channel to at least one sample chamber (4);
- allowing the sample solution (6) to contact with the mixture (7) so as to at least partially dissolve the mixture (7) in the sample solution (6);
- detecting light emitted from the sample chamber (4); and
- determining from the detected light a varying emission spectrum of the emitted light resulting from the varying extracellular environmental conditions inside the sample chamber, wherein detecting light emitted from the sample chamber (4) preferably comprises:
- directing a light beam from a light source to the sample solution (6) inside the sample chamber (4);
- detecting light emitted from the sample chamber (4), the emitted light being caused by luminescence in the sample solution inside the sample chamber;
and/or further comprising, before allowing the sample solution (6) to contact with the mixture (7):
monitoring extracellular environmental conditions in the at least one sample chamber (4), for collecting data corresponding to the emission spectrum of the at least one chemical sensor (9),
the method optionally comprising periodically or substantially continuously monitoring extracellular environmental conditions in the at least one sample chamber (4), in real-time during the allowing the sample solution (6) to contact with the mixture (7) and/or wherein a capillary flow occurs during 0.1-10 seconds and/or wherein the monitoring is performed during 1-8 hours, preferably 3-5 hours; and/or optionally comprising at least partially dissolving the mixture (7) comprises dissolving at least 40% or 50%, preferably at least 70%, more preferably at least 90% of the mixture (7), and most preferably comprises dissolving substantially all of the mixture (7) and/or wherein the method comprises allowing the mixture to substantially dissolve or diffuse into the sample solution (6) within 10 minutes, preferably within 5 minutes, even more preferably within 1 minute, after loading of the at least one sample solution into the microstructure (2) and/or enhancing the mixing of the sample solution (6) with the mixture (7) by applying a magnetic field within the at least one sample chamber (4) to move a magnetic elements (12) present in the matrix (7) and/or further comprising enhancing the mixing of the sample solution (6) with the mixture (7) by applying ultrasound to the sample chamber (4).

15. Use of a microfluidic device according to any one of the claims 1-12 for detecting the susceptibility of a microbe (18) present in a sample solution (6) to a predetermined drug (17) and/or for detecting the relative susceptibility of a microbe (18) present in a sample solution (6) to a plurality of different predetermined drugs (17).
